# EUROPEAN PATENT APPLICATION

(11) **EP 4 049 582 A1**
(43) Date of publication of application: **31.08.2022**
(21) Application number: 22168445.9
(22) Date of filing: 12.12.2018
(51) Int. Cl.: A61B 5/00

(54) **NASAL AND ORAL RESPIRATION SENSOR**

(30) Priority: 12.12.2017 US 201762597870 P
(62) Divisional of application: 18836744.5
(71) Applicant: Vyaire Medical, Inc., Mettawa, Illinois 60045 (US)
(72) Inventor: HAVERI, Heikki, 03150 Huhmari (FI); RANTA, Janne, 02710 Espoo (FI)
(74) Representative: Epping - Hermann - Fischer

(57) **Abstract**

An apparatus having a support structure, including two nasal flow passages aligned with one another and with respect to a nasal respiratory flow direction, and an oral flow passage disposed transverse to the two nasal flow passages, along an oral respiratory flow direction, the nasal flow passages and oral flow passages having thermistors to monitor a patient's respiration, and the apparatus having a thermistor for monitoring ambient conditions and an accelerometer for monitoring movement of the apparatus. The apparatus also detecting and distinguishing between oral and individual nasal air flows, and integration of the apparatus and monitored data with a network.

## Description

### CROSS-REFERENCE TO RELATED APPLICATIONS

This application claims the benefit of U.S. Patent Application No. 62/597,870, filed on December 12, 2017, the entirety of which is incorporated herein by reference.

### BACKGROUND

The present disclosure relates generally to medical sensors. More particularly, the present disclosure relates to respiration sensors for a continuous, long-lasting monitoring of an individual or patient, including measuring and analyzing respiratory condition and movement of the person.

The respiration of a person may be monitored for various reasons. For example, knowledge about a patient's respiration may assist a caregiver in assessing the patient's stability during surgery and recovery thereafter. Knowledge about a person's respiration can also assist with therapy related to sleeping.

Many approaches to respiration sensors involve cumbersome devices that can obstruct a patient's respiratory passages. In many applications, the patient is unconscious or semi-conscious and there is a challenge to fix a respiration sensor in place for an extended period of time. Accordingly, in many of the existing systems a nurse is required to frequently check the patient for sensor placement or inadvertent sensor movement. Moreover, due to the physiognomy of the human respiratory passages, many devices tend to produce confused readings relative to either of a patient's nostrils and mouth, and fail to clearly distinguish and provide differentiated data for inspiration and exhalation steps.

### SUMMARY

The invention is defined by the claims. In the following, parts of the description and drawings referring to embodiments which are not covered by the claims are not presented as embodiments of the invention, but as examples useful for understanding the invention.

In the field of medical care for patients with respiratory dysfunction, it is highly desirable to provide continuous, real-time measurement of the patient's respiratory cycles. In the measurement of respiratory cycles from patients, one of the challenges is to clearly distinguish between inhalation and exhalation cycles. The complication is compounded by the human physiognomy, which places nasal and oral flows (in and out of the patient) in close proximity to each other, thereby increasing the possibility of flow mix, turbulence, and stagnation in some places.

An aspect of the present disclosure provides, but is not limited to, a respiration sensor for monitoring and analysis of an individual or patient's respiratory condition and cycle, monitoring and analysis to ensure a respiration sensor is positioned as intended, detecting movement of a person using a respiration sensor, detecting and distinguishing between oral and individual nasal air flows, and integration of a respiration sensor and data with a network.

In some embodiments, the present disclosure provides a respiration sensor comprising: a housing having a nasal flow passage that extends therethrough, wherein the nasal flow passage is disposed approximately parallel with a nasal respiratory flow direction; and an electronics board comprising a nasal thermistor, the electronics board coupled to the housing such that the nasal thermistor is positioned into the nasal flow passage.

In some embodiments, a respiration sensor is disclosed, the respiration sensor comprising: a housing having a first nasal flow passage and a second nasal flow passage that extend therethrough, wherein the nasal flow passages are disposed in parallel to one another with respect to a nasal respiratory flow direction; and an electronics board comprising a first nasal thermistor and a second nasal thermistor, the electronics board coupled to the housing such that the first and second nasal thermistors are positioned into each of the first and second nasal flow passages, respectively.

In some embodiments, the present disclosure provides a respiration sensor comprising: one or more thermistors configured to detect at least one of an inspiratory temperature, an expiratory temperature, an ambient temperature adjacent the respiratory sensor, or a temperature of a patient's skin engaged against the respiratory sensor; an accelerometer configured to detect at least one of a movement of the patient, a position of the patient, a heart rate, or a respiration rate; and an electronics board coupled to the one or more thermistors and the one or more thermistors.

In some embodiments, the present disclosure provides a system, comprising: a server having a memory storing commands, and a processor configured to execute the commands to: receive, from a hub, a data indicative of a respiratory condition of a patient; transfer the data into a memory in a remote server; provide the data to a mobile computer device, upon request; and instruct the mobile computer device to graphically display the data, wherein the data comprises a temperature value from at least one of two nasal flow passages, a temperature value from an oral flow passage, a temperature value of a patient's skin surface, and a temperature value of a patient's environment.

In some embodiments of the present disclosure, a method is disclosed, the method comprising: receiving, from a hub, a data indicative of a respiratory condition of a patient; transferring the data into a memory in a remote server; providing the data to a monitor, upon request; and instructing the monitor to graphically display the data, wherein the data comprises a temperature value from at least one of two nasal flow passages, a temperature value from an oral flow passage, a temperature value of a patient's skin surface, and a temperature value of a patient's environment.

In some embodiments a respiration sensor system is disclosed, the respiration sensor system comprising: a respiration sensor comprising a housing having a nasal flow passage that extends therethrough, wherein the nasal flow passage is aligned with a nasal respiratory flow direction, and an electronics board comprising a nasal thermistor, the electronics board coupled to the housing such that the nasal thermistor is positioned into the nasal flow passage; and a hub configured to move data between the respiration sensor and a network.

Additional features and advantages of the subject technology will be set forth in the description below, and in part will be apparent from the description, or may be learned by practice of the subject technology. The advantages of the subject technology will be realized and attained by the structure particularly pointed out in the written description and embodiments hereof as well as the appended drawings.

It is to be understood that both the foregoing general description and the following detailed description are exemplary and explanatory and are intended to provide further explanation of the subject technology.

### BRIEF DESCRIPTION OF THE DRAWINGS

Various features of illustrative embodiments of the inventions are described below with reference to the drawings. The illustrated embodiments are intended to illustrate, but not to limit, the inventions. The drawings contain the following figures.
FIG. 1 illustrates a front perspective view of a respiration sensor placed on a patient's head, according to some embodiments.
FIG. 2A illustrates a side plan view of a gas flow exiting from a patient's nasal cavity, according to some embodiments.
FIG. 2B illustrates a side plan view of a gas flow exiting from a patient's oral cavity, according to some embodiments.
FIG. 3 illustrates a front plan view of a gas flow exiting from a patient's nasal cavity, according to some embodiments.
FIG. 4 illustrates a front perspective view of nasal respiration flows and oral respiration flows in a patient, according to some embodiments.
FIG. 5 illustrates a front perspective view of a respiration sensor including nasal flow passages and oral flow passages, according to some embodiments.
FIG. 6 illustrates perspective detail views of nasal flow passages and oral flow passages of a respiration sensor, according to some embodiments.
FIG. 7 illustrates a cross-sectional view of a laminar respiration flow relative to a thermistor in a respiration sensor, according to some embodiments.
FIG. 8 illustrates a schematic view of a flow passage of a respiration sensor, according to some embodiments.
FIGS. 9A and 9B illustrate front and back perspective views of a respiration sensor, according to some embodiments.
FIG. 10 illustrates a front perspective view of a respiration sensor placed on a patient's head, according to some embodiments.
FIG. 11 illustrates a front perspective view of a respiration sensor, according to some embodiments.
FIG. 12 illustrates a bottom perspective view of the respiration sensor of FIG. 11.
FIG. 13 illustrates a front perspective detail view of a respiration sensor, according to some embodiments.
FIG. 14 illustrates a front perspective cross-sectional view of the respiration sensor of FIG. 11.
FIG. 15 illustrates a schematic view of a nasal respiration flow and a nasal flow guide, according to some embodiments.
FIG. 16 illustrates a schematic view of a nasal flow guide, according to some embodiments.
FIG. 17 illustrates a back perspective view of a respiration sensor, according to some embodiments.
FIG. 18 illustrates a schematic view of a respiration flow through a cavity of a respiration sensor, according to some embodiments.
FIG. 19 illustrates a schematic view of turbulent respiration gas flow through a cavity of a respiration sensor, according to some embodiments.
FIG. 20 illustrates a graph showing turbulent noise flow during expiration, according to some embodiments.
FIG. 21A and 21B illustrate front and side plan views of exit angles for a gas flow exiting from a patient's nasal cavity, according to some embodiments.
FIG. 22A and 22B illustrate front and side schematic views of a position of an oral cavity relative to a patient, according to some embodiments.
FIG. 23 illustrates a front perspective view of a respiration sensor, according to some embodiments.
FIG. 24 illustrates a graph showing measurements for the respiration sensor of FIG. 23 for different patients, according to some embodiments.
FIGS. 25A and 25B illustrates a front plan views of positions of an oral cavity for a respiration sensor relative to a mouth of different patients, according to some embodiments.
FIG. 26 illustrates a front perspective view of a respiration sensor having a strap, according to some embodiments.
FIG. 27 illustrates a front perspective view of a respiration sensor having a band, according to some embodiments.
FIG. 28 illustrates a front perspective exploded view of a respiration sensor, according to some embodiments.
FIG. 29 illustrates a top perspective detail view of an electronics board and frame of a respiration sensor, according to some embodiments.
FIG. 30 illustrates a top perspective view of an electronics board of a respiration sensor, according to some embodiments.
FIG. 31 illustrates a top perspective detail view of the electronics board of FIG. 30, according to some embodiments.
FIG. 32 illustrates a bottom perspective view of the electronics board of FIG. 30, according to some embodiments.
FIG. 33 illustrates a top perspective view of the electronics board of FIG. 30 coupled with a frame and a battery, according to some embodiments.
FIG. 34 illustrates a block diagram of an electronics board of a respiration sensor, according to some embodiments.
FIG. 35 illustrates another block diagram of an electronics board of a respiration sensor, according to some embodiments.
FIG. 36 illustrates a respiration sensor detection state table for determining the respiration sensor placement and function, according to some embodiments.
FIG. 37 illustrates a graph showing breathing during changes in ambient air temperature, according to some embodiments.
FIG. 38 illustrates a graph showing breathing during conducting temperature changes, according to some embodiments.
FIG. 39 illustrates a respiration sensor in use on a patient transitioning from a seated position, to a moving position, to a fallen position, according to some embodiments.
FIG. 40 illustrates a front plan view of a heart and directions of blood circulation therethrough.
FIG. 41 illustrates a front plan view of a respiration sensor including an accelerometer for detecting body movement of a patient utilizing the respiration sensor, according to some embodiments.
FIG. 42 illustrates a front perspective exploded view of a respiration sensor having EtCO2 sensitive surfaces, according to some embodiments.
FIG. 43 illustrates a schematic view of a respiration monitoring system, according to some embodiments.
FIG. 44 illustrates a front perspective view of a respiration sensor coupled to a patient and a hub adjacent to the patient according to some embodiments.
FIG. 45 illustrates a front perspective view of a respiration sensor and hub coupled to a patient, according to some embodiments.
FIG. 46 illustrates perspective detail views of an interaction between a respiration sensor and a hub in a respiration monitoring system, according to some embodiments.
FIG. 47 illustrates a front perspective view of a respiration sensor and hub coupled with a headdress, according to some embodiments.
FIG. 48 illustrates a side view of a respiration sensor and hub coupled with another headdress, according to some embodiments.
FIG. 49 illustrates a front perspective view of a smartphone as a monitor for a respiration monitoring system, according to some embodiments.
FIG. 50 illustrates a front perspective view of a central station as another monitor for a respiration monitoring system, according to some embodiments.

In the figures, elements having the same or similar reference numeral have the same or similar functionality or configuration, unless expressly stated otherwise.

### DETAILED DESCRIPTION

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings, and detailed description are to be regarded as illustrative in nature and not as restrictive.

The detailed description set forth below is intended as a description of various configurations of the subject technology and is not intended to represent the only configurations in which the subject technology may be practiced. The appended drawings are incorporated herein and constitute a part of the detailed description. The detailed description includes specific details for the purpose of providing a thorough understanding of the subject technology. It will be apparent, however, to one ordinarily skilled in the art that the embodiments of the present disclosure may be practiced without some of these specific details. In other instances, well-known structures and components are shown in block diagram form in order to avoid obscuring the concepts of the subject technology, or have not been shown in detail so as not to obscure the disclosure. Like components are labeled with similar element numbers for ease of understanding.

In accordance with at least some embodiments disclosed herein is respiration sensor that can: monitor nasal and oral respiration gas flow; monitor patient and ambient conditions; monitor movement of the respiration sensor; distinguish between oral and nasal air flow, and between left and right nasal air flow. The respiration sensor can identify and analyze thermal transfer distinction between inhalation and exhalation gases to provide a clear pattern of the respiratory cycle.

In at least some embodiments disclosed herein, any of nasal and oral respiration gas flow, heart rate, respiration rate or cycle, and movement of the respiration sensor and patient are determined. Embodiments of the present disclosure can send and receive data related to the monitoring and analysis by the respiration sensor; indicate a patient's condition or position; and provide a signal or alarm corresponding to specific conditions. In some embodiments, wireless communication techniques are utilized to provide ubiquitous solutions for respiratory sensing of patients in hospitals, treatment facilities, home-care situations, and the like.

### I. Embodiments of Respiratory Sensors

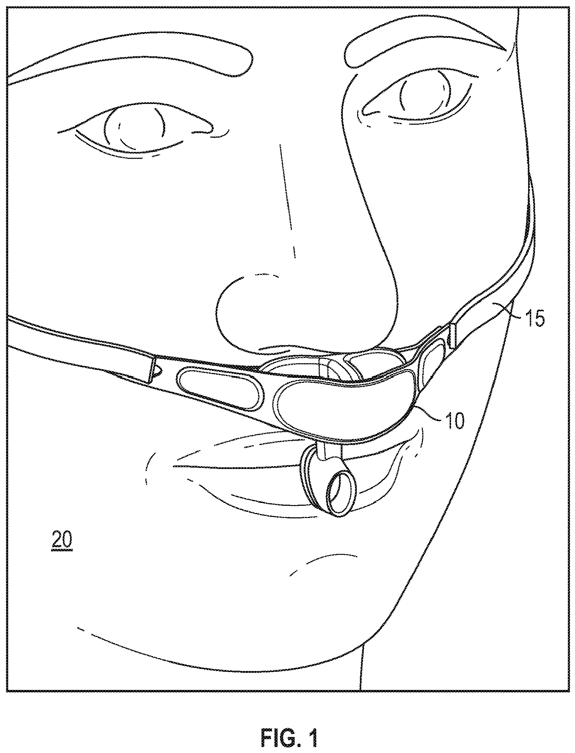

FIG. 1 illustrates a respiration sensor 10 placed on a patient's head 20, according to some embodiments. The respiration sensor 10 is positioned on patient's face between the mouth and nose to measure nasal and oral breathing gas flow. The gas flow measurement is based on measuring temperature differences between inspiratory and expiratory gas flows. Patient's skin and ambient air temperatures can also be measured to verify that the respiration sensor 10 is placed appropriately against the patient. Some embodiments, later described, include other sensors, such as capacitive sensors or detectors and accelerometers to ensure that respiration sensor 10 has not fallen out of place, and that respiration sensor 10 is making proper contact with the patient's physiognomy. A securement string or strap 15 helps maintain the position of respiration sensor 10 relative to the patient's physiognomy.

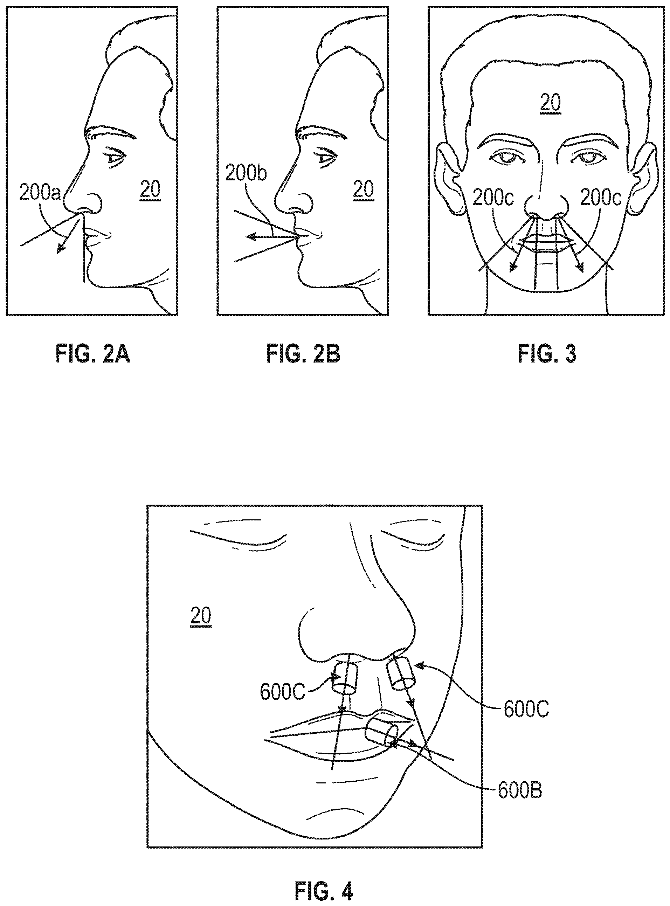

FIGS. 2A and 3 illustrate regions 200a, 200c for a gas flow exiting from a patient's nasal cavities, and FIG. 2B illustrates regions 200b for a gas flow exiting from a patient's oral cavity, according to some embodiments. Experiments show that breathing gas flow exits nasal and oral cavities in different regions between different subjects. Accordingly, embodiments of a respiration sensor as disclosed herein include a geometry that may separate each of the different flows through the regions 200a, 200b, 200c to provide a more accurate measure of the respiratory cycles of a patient. Accordingly, a precise determination of the positioning of the respiration sensor 100a, 100b relative to the patient's face is highly desirable.

FIG. 4 illustrates a portion of nasal respiration flow 600C and oral respiration flow 600B for a patient 20, according to some embodiments. Sensor cavities of the respiration sensor capture nasal and oral breathing gas flow from the patient. The sensor cavities are positioned parallel to the average direction of that specific flow to maintain flow as laminar as possible inside the cavity. Thus, nasal sensor cavities are positioned parallel to each other between the nose and mouth, but also parallel to upper lip. More advantageously, nasal sensor cavities slightly diverge past the middle part of the mouth and upper lip into the average direction of nasal breathing gas flows. An oral sensor cavity is positioned transverse to the nasal cavities, outwards from the mouth. In some embodiments, the oral sensor cavity and the nasal sensor cavities are positioned relative to each other so that a direction of oral respiration flow 600B through the oral cavity is transverse relative to a direction of nasal respiration flow 600C through any of the nasal sensor cavities. Sensor cavities are also smooth and straight, or more advantageously slightly tapered, to capture flow from a larger area, since any turn or sudden change in the cross-section of cavity along the flow path generate turbulences that mix inspiratory and expiratory air flow phases degrading the measurement speed, accuracy and response time.

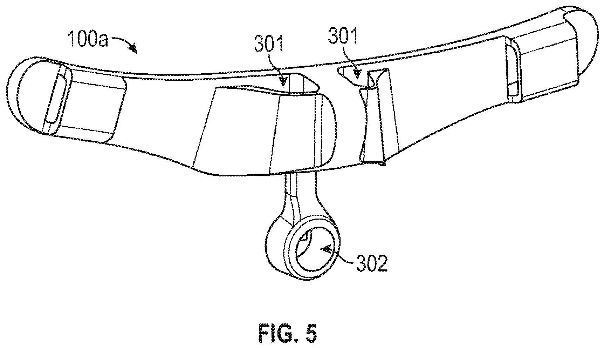

FIG. 5 illustrates a respiration sensor 100a, for example, including nasal flow passages 301 and an oral flow passage 302. The nasal respiration flow exiting a patient's nasal cavity, e.g., gas flow regions 200a, 200c, can be captured and guided by a nasal passage 301 parallel to average direction of nasal respiration flow 600C. Similarly, the oral respiration flow exiting a patient's mouth, e.g., gas flow region 200b, can be captured and guided by the oral cavity 302 parallel to direction of the oral respiration flow 600B. By providing a sensing element inside of each of the different flow passages 301 and 302, the respiration sensor 100a may accurately determine a respiration flow before the nasal flow and the oral flows are mixed together adjacent the patient's upper lip.

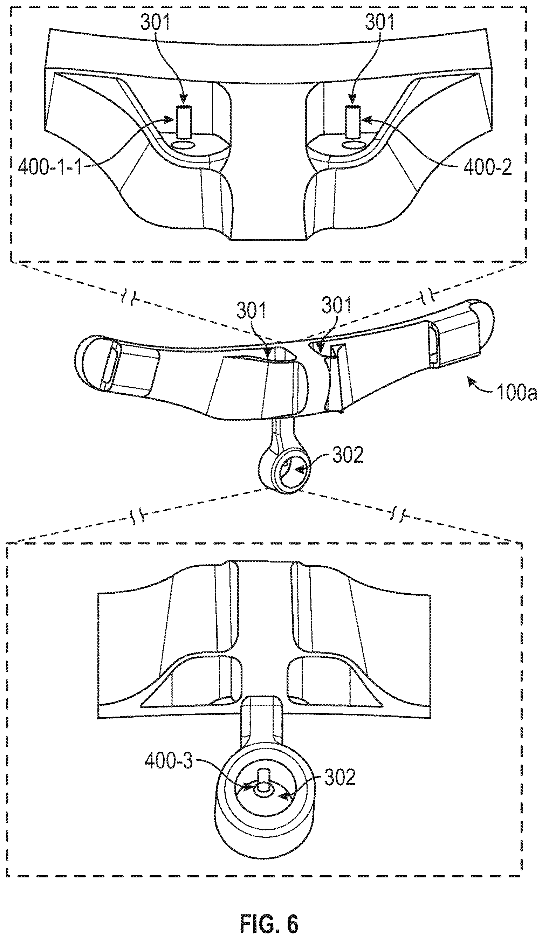

FIG. 6 illustrates the respiration sensor 100a, with portions thereof shown in detail views, including detail views of the nasal flow passages 301 and the oral flow passage 302. The respiration sensor 100a includes thermistors 400-1, 400-2, 400-3 for sensing inhalation and exhalation flows. A nasal respiratory flow of a patient can be captured by the nasal passages 301 and measured with a first and second nasal thermistors 400-1, 400-2 therein. An oral respiratory flow of the patient can be captured by the oral cavity 302 and measured with thermistor 400-3 therein. The resistance of each thermistor changes proportionally to flowing gas heating or cooling down the thermistor, e.g., during inspiration and expiration.

Moreover, the nasal flow passages 301 are separated from each other such that nasal thermistors 400-1 and 400-2 may separately identify and measure the respiration flow associated with each of the patient's nostrils. By separately identifying respiration flow associated with each of the patient's nostrils, potential respiratory conditions or patient's positions can be determined. For example, a blockage of a nasal passage or the respiration device can be identified and corrected.

In some embodiments, oral thermistor 400-3 is placed on a plane that is transverse or substantially perpendicular to nasal thermistors 400-1, 400-2. This geometry also enables an accurate and independent measurement between each of the thermistors 400-1, 400-2, 400-3, avoiding any mixing or turbulent area.

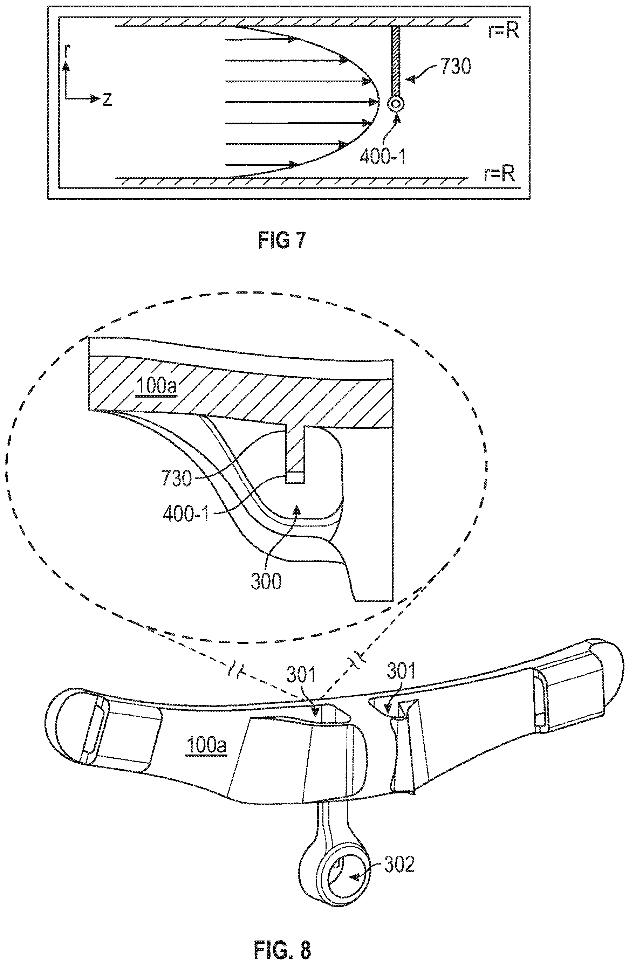

Referring to FIGS. 7 and 8, the thermistors 400-1, 400-2, 400-3 can be located approximately in the middle of its corresponding sensor cavity to maximize accuracy and sensitivity to gas flows. To position the thermistor 400-1, 400-2, 400-3 in the middle of a corresponding sensor cavity, the thermistor 400-1, 400-2, 400-3 is coupled to a tip portion of a thin support structure 730. The support structure can have a proximal portion coupled to an electronics board and a distal portion transverse to a plane defined by the top of the electronics board, wherein the distal portion of the support structure extends into a nasal flow passage. In some embodiments, the respiration sensor 100a has a structure and geometry that separates the nasal flow from each nostril separately, to provide a more accurate and detailed picture of the patient's respiratory condition.

FIG. 7 illustrates a cross-section of a laminar respiration flow of a patient through a sensor cavity. Laminar flow speed distribution in a tube is parabolic, thus the speed is maximum at a point approximately in the middle of the tube. The respiration flow is illustrated relative to thermistor 400-1 of a respiration sensor 100a, however, the present disclosure can apply to any thermistor 400-1, 400-2, 400-3. By placing thermistor 400-1 as close as possible to the middle of the flow cavity in the respiration sensor 100a, for example, a more accurate measurement is expected, as the velocity of the gas flow is highest at the center of the flow cavity. Accordingly, it is expected that a temperature differential between inhalation and exhalation be highest at the middle point of the flow cavity. Moreover, the convection or radiated thermal energy from surrounding structures is minimized when a thermistor 400-1, 400-2, 400-3 is located into the middle of cavity by a support structure 730.

FIG. 8 illustrates the respiration sensor 100a, with a portion thereof shown in a cross-sectional detail view. The cross-sectional view illustrates a support structure 730 extending into the nasal flow passage 301, and the thermistor 400-1 positioned at a distal end portion of the support structure 730. It should be understood that the present disclosure, including support structures, can apply to any of the thermistors 400-1, 400-2, 400-3 and flow passages 301, 302.

The support structure 730 extends from a portion of the respiration sensor 100a into the nasal flow passage 301. It should be understood that the support structure 730 can extend partially into a flow passage 301, 302. For example, the support structure 730 can extend into a mid-portion of at least one of the two nasal flow passages 301. In some embodiments of the present disclosure, the support structure 730 extends beyond or across the respective flow passage 301, 302. The support structure 730 can comprise a cantilevered structure that extends into a respective flow passage 301, 302. However, in some embodiments, the support structure 730 can comprise an arch structure partially extending away from an inner surface of the flow passage 301, 302 toward the thermistor 400-1, and partially extending from the thermistor 400-1 toward the inner surface of the flow cavity. In some embodiments, the support structure 730 and the thermistor 400-1 can extend across inner surfaces of the flow cavity.

The respiration sensor 100a includes walls having an inner surface forming the sensor cavities. The walls of the cavity extend around at least a portion of the thermistors 400-1, 400-2, 400-3. The walls protect the sensitive thermistors 400-1, 400-2, 400-3 from various disturbing ambient gas flows causing error to measured breathing gas flow signal, for example, a caregiver being able to touch or breathe into thermistors or air conditioning in proximity to the thermistor 400-1, 400-2, 400-3. In addition, the walls forming the cavities also protect small, mechanically sensitive thermistors from various mechanical forces, stresses, and shocks, such as touching etc.

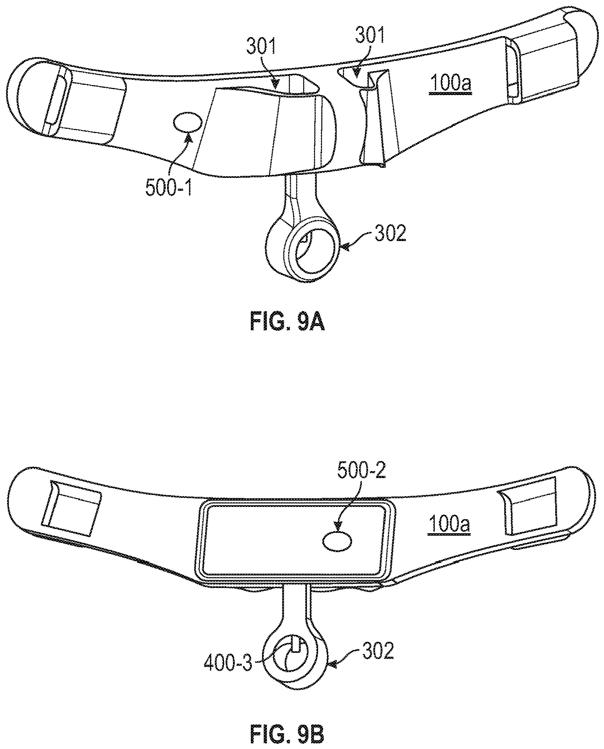

FIGS. 9A and 9B illustrate the respiration sensor 100a, for example, including thermistors 500-1, 500-2 for sensing the positioning of the sensor relative to a patient's physiognomy, according to some embodiments. An ambient thermistor 500-1 can be positioned along a front side of the respiration sensor 100a, adjacent a portion of the respiration sensor 100a that faces away from the patient when the respiration sensor 100a is worn by a patient. Similarly, a skin thermistor 500-2 can be positioned along a back side of the respiration sensor 100a, adj acent a portion of the respiration sensor 100a that faces toward the patient when the respiration sensor 100a is worn by a patient. In some embodiments, when the respiration sensor 100a is worn by a patient, the thermistor 500-1 is distal to the patient's face, and the thermistor 500-2 is proximal to the patient's upper lip and engaged against the patient's skin.

The respiration sensor 100a can include a passage or cavity along any of the front side or the back side thereof. The thermistor 500-1 can be positioned in a cavity along the front side of the respiration sensor 100a to measure ambient air temperature. The thermistor 500-2 can be positioned in a cavity along the back side of the respiration sensor 100a to measure the temperature of patient's skin.

In some instances, thermistor 500-2 can detect when the sensor 100a is properly positioned on the patient while thermistor 500-1 can detect the temperature of ambient air. Comparison of temperatures from 500-1 and 500-2 can be used to indicate a patient condition or proper positioning and function of the sensor 100a, for example. In some embodiments, when thermistors 500-1 and 500-2 detect the same temperature, it may be assumed that respiration sensor 100a is likely not attached to the patient, or that the patient's temperature is the same as the ambient temperature, which may indicate a hazardous health condition.

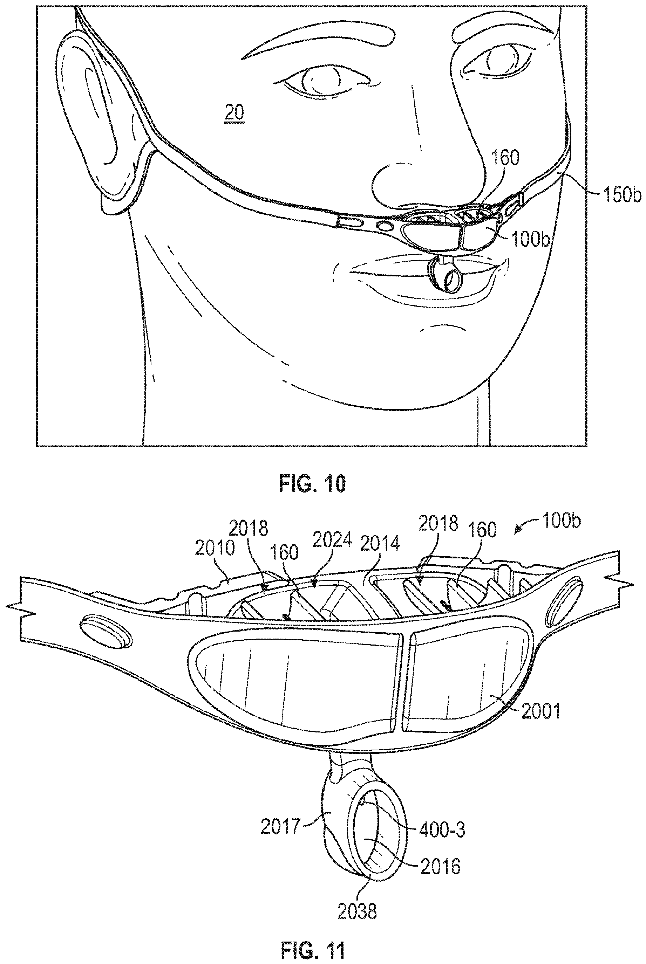

FIG. 10 illustrates another embodiment of a respiration sensor 100b, which is substantially similar to respiration sensor 100a. Respiration sensor 100b is also placed on a patient's face between the mouth and nose to measure nasal and oral breathing gas flows. Much like the respiration sensor 100a, the measurement is based on measuring temperature differences between inspiratory and expiratory gas flows. The patient's skin temperature and the ambient air temperature can also be measured to verify or detect that the respiration sensor 100b is placed appropriately with respect to the patient's nasal and oral breathing gas flows and to the patient's upper lip.

Some embodiments described herein include other sensors, such as capacitive detectors or sensors to detect whether the respiration sensor 100b is making proper contact with the patient's physiognomy and accelerometers to detect movement and position of the respiration sensor 100b to ensure, for example, that the respiration sensor 100b has not fallen out of place, that the patient has not fallen down, or that the orientation of the patient's head is not obstructing the nasal and oral breathing gas flows (e.g., patient's face is downward towards pillow or bed).

A string or strap 150b helps maintain the position of the respiration sensor 100b relative to the patient's physiognomy. According to some embodiments, the respiration sensor 100b can include a nasal flow guide 160 to concentrate and provide laminar inspiratory and expiratory gas flows through the respiration sensor 100b.

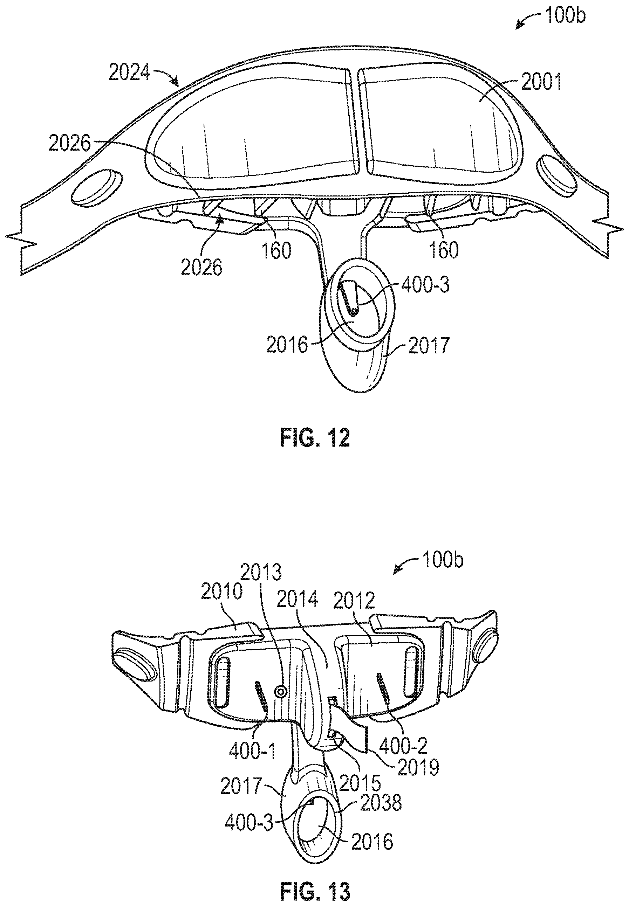

FIGS. 11 and 12 illustrate the respiration sensor 100b having a housing 2001, a base 2010, and a shroud 2012. The shroud 2012 is positioned between the housing 2001 and the base 2010 to form at least a portion of a cavity. The respiration sensor 100b includes nasal flow passages 2018, which are similar to nasal flow passages 301, and an oral flow passage 2016, which is similar to oral flow passages 302 of respiration sensor 100a. The nasal flow passages 2018 extend from a top portion to a bottom portion of the respiration sensor 100b. In use, a nasal respiration flow from a patient's nose can move between the nasal inlet 2024 and the nasal outlet 2026 of each of the nasal flow passages 2018. The nasal inlet 2024 of each of the nasal flow passages 2018 is where the breathing gas flows into the respiration sensor 100b during expiration. The nasal outlet 2026 of each of the nasal flow passages 2018 is where the ambient air flows into the respiration sensor 100b during inspiration.

The shroud 2012 includes a battery frame 2014, which extends away from a front surface of the shroud 2012. The battery frame 2014 encloses a battery, securing it to the base 2010 and divides the area between the shroud 2012 and the housing 2001 into two distinct nasal flow passages 2018, such that the nasal thermistor 400-1 is centrally disposed in one of the nasal flow passages 2018 and the nasal thermistor 400-2 is centrally disposed in the other one of the nasal flow passages 2018. The battery frame 2014 is disposed substantially centrally on the respiration sensor 100b and is arranged to be positioned under the septum of a patient's nose when the respiration sensor 100b is placed on or attached adjacent to the upper lip of the patient.

Housing 2001 can be made of a paper battery engineered to use a spacer formed largely of cellulose that makes paper batteries flexible and environmentally-friendly. The functioning is similar to conventional chemical batteries with the important difference that they are non-corrosive and do not require extensive housing, but can function as housing.

An oral shroud 2017 extends from the shroud 2012, and includes a passage through a distal portion thereof. The passage forms an oral flow passage 2016 having a thermistor 400-3 positioned therein. The oral flow passage 2016 is arranged such that the oral thermistor 400-3 is centrally disposed within the oral flow passage 2016. In use, an oral respiration flow from a patient's mouth can move between the oral inlet 3036 and the oral outlet 2038.

FIG. 13 illustrates an embodiment the respiration sensor 100b, showing the base 2010 and the shroud 2012, with the housing 2001 omitted for clarity. The shroud 2012 encloses an electronics board, securing it to the base 2010. The thermistors 400-1, extend from the electronics board through the shroud 2012. The thermistors 400-1, 400-2 are oriented such that a distal portion of the thermistors 400-1, 400-2 extend into a space forming the nasal cavities 1301 when the shroud 2012 and the housing 2001 are coupled together.

The respiration sensor 100b can include a light-emitting diode (LED) 2013, which is visible through the shroud 2012. The LED 2013 can provide a confirmation or an indication of status. For example, the LED 2013 can indicate when the respiration sensor 100b is paired with another device. In some embodiments, the LED 2013 can indicate any of a charged or low battery, an indication that the respiration sensor 100b is functioning as intended, or an indication that there is a detected problem with the respiration sensor 100b. The LED 2013 can be used to indicate the location of the patient for example in hospital PACU where there are many patients, respiration sensors and monitoring devices in the same room. The LED 2013 can be turned on or display a series of blinks from the monitoring device to indicate the location of the patient and the connected respiration sensor. This may be important to ensure that a caregiver is looking at the correct monitoring device connected to patient and the respiration sensor. It should be understood that any embodiment of the respiration sensor, such as respiration sensor 100a, 100b, can include an LED 2013.

In some embodiments, a spacer 2019 can be positioned between the battery and a battery contact. The spacer 2019 can maintain the battery contact spaced apart from the battery, thereby preventing electrical conduction therebetween. The spacer 2019 can prevent discharge of the battery before the respiration sensor 100b is intended to be used. When the respiration sensor 100b is intended to be used, the spacer 2019 can be removed or separated from the respiration sensor 100b. In some embodiments, the respiration sensor 100b can comprise an opening or passage 2015 that extends between the battery and an outer surface of the housing 2001 or the shroud 2012. The spacer 2019 can be moved through the passage 2015 to separate the spacer 2019 from the respiration sensor 100b. In some embodiments, the spacer 2019 may comprise a plastic material in the form or a strip or tape.

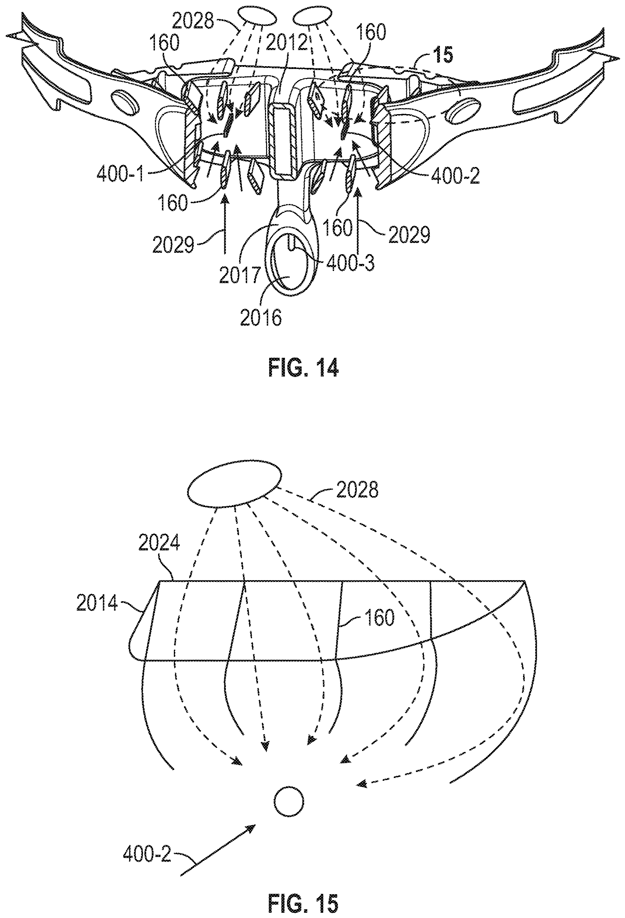

FIG. 14 illustrates an embodiment the respiration sensor 100b, showing the base 2010, the shroud 2012, and the flow guides 160, with a portion of the housing 2001 and other features omitted for clarity. At least one nasal flow guide 160 is disposed in each of the nasal flow passages 2018 and extends between the shroud 2012 and the housing 2001, as shown in at least FIGS. 11 and 12.

In some embodiments, at least one nasal flow guide 160 is disposed proximate a nasal inlet 2024 of each of the nasal flow passages 2018 and at least one nasal flow guide 160 is disposed within each of the nasal flow passages 2018. A nasal flow guide 160 can be positioned in a nasal flow passage, proximate any of the nasal inlet 2024 and the nasal outlet 2026. The nasal flow guide 160 is aligned relative to the nasal thermistor 400-1 or 400-2 to direct a flow of gas toward relative to the nasal thermistor 400-1 or 400-2.

FIGS. 14 and 15 illustrates flow of gases relative to the respiration sensor 100b, a patient's nares, and the ambient environment. Arrows 2028 illustrate a portion of nasal respiration flow from a patient's nares toward the nasal thermistor 400-1, 400-2 during expiration, and arrows 2029 illustrate a portion of ambient gas directed from the ambient environment toward the nasal thermistor 400-1, 400-2 during inspiration.

In more detail, during expiration, the at least one nasal flow guide 160, disposed proximate the nasal inlet 2024 guides the breathing gas flow through the nasal flow passages 2018 of the respiration sensor 100b and concentrates the breathing gas flow toward each of the nasal thermistors 400-1, 400-2 while maintaining the breathing gas flow laminar as it passes each of the nasal thermistors 400-1, 400-2 to minimize turbulent noise. Similarly, during inspiration, the at least one nasal flow guide 160 disposed proximate the nasal outlet 2026 guides the ambient air flow through the nasal flow passages 2018 of the respiration sensor 100b and concentrates the ambient air flow toward each of the nasal thermistors 400-1, 400-2 while maintaining the ambient air flow laminar as it passes each of the nasal thermistors 400-1, 400-2 to minimize turbulent noise.

The at least one nasal flow guide 160 can prevent undesired objects from entering the nasal flow passages 2018 and disturbing or breaking the nasal thermistors 400-1, 400-2 and/or their associated support structures. The at least one nasal flow guide 160 can also form an air gap around the nasal thermistors 400-1, 400-2 with respect to the housing 2001 and the at least one nasal flow guide 160, which prevents electro static discharge (ESD) from entering the electronics board 300 via the nasal thermistors 400-1, 400-2 and their associated support structures.

In some embodiments, the at least one nasal flow guide 160 includes a thickness that is less than 1 mm and a height that is more than 2 mm. In some embodiments, two or four nasal flow guides 160 are disposed within each of the nasal flow passages 2018 proximate the nasal inlet 2024 and/or two or four nasal flow guides are disposed within each of the nasal flow passages 2018 proximate the nasal outlet 2026. In some embodiments, the number of nasal flow guides 160 does not exceed five to allow for proper gas flow through the nasal flow passages 2018.

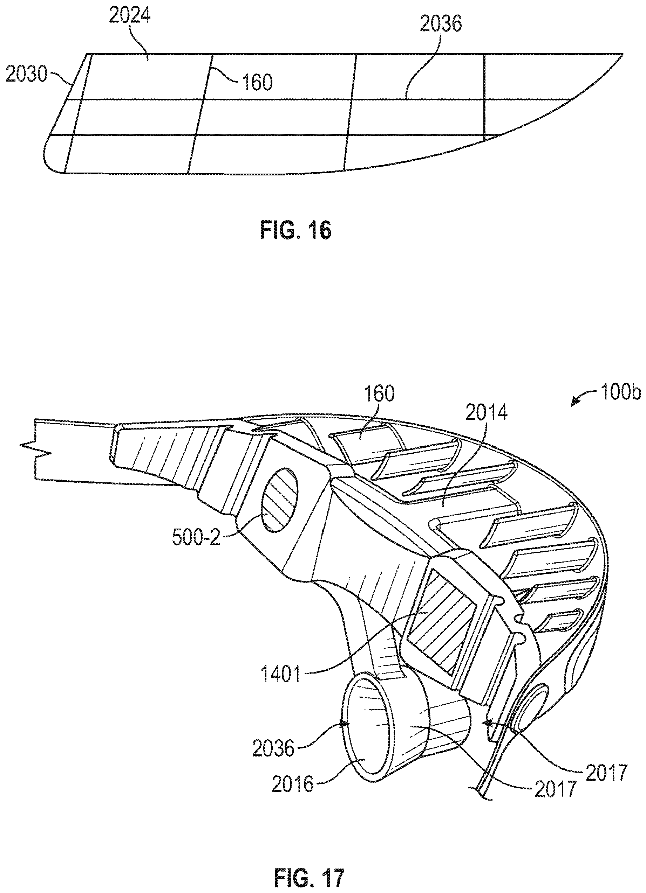

FIG. 16 illustrates a schematic view of a nasal respiration flow guide grid 2030. The flow guide grid 2030 can function similarly to flow guide 160, wherein a flow of gas through a cavity of the respiration sensor 100b is directed by the flow guide grid 2030. The flow guide grid 2030 can have walls which intersect and are transverse relative to each other. In some embodiments, a flow guide grid 2030 is disposed proximate the nasal inlet 2024 and a flow guide grid 2030 is disposed proximate the nasal outlet 2026 of each nasal cavity of the nasal flow passages 2018.

Additional sensors of a respiration sensor 100b are illustrated in the back, perspective view of the respiration sensor 100b in FIG. 17. The respiration sensor 100b includes a thermistor 500-2 and a sensor 1401 located on the back portion of the respiration sensor 100b. The thermistor 500-2 can provide temperature information regarding the patient or an ambient environment adjacent to the back portion of the respiration sensor 100b. The sensor 1401 is a capacitive plate, which can engage against the patient. The sensor 1401 can engage against a region between a patient's upper lip and nose, e.g., an area including the philtrum, and provide information to determine a location of the respiration sensor 100b relative to the patient's face.

### II. Gas Flow Through Respiration Sensors

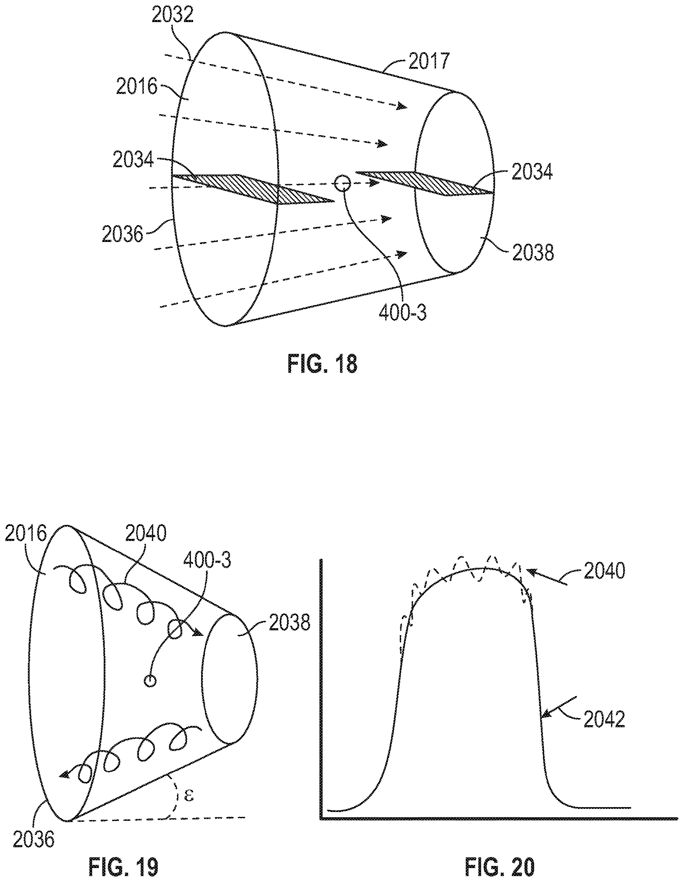

Referring to FIGS. 17 and 18, an oral shroud 2017 of the respiration sensor 100b can have a cross-sectional area that tapers along a portion thereof or relative to an oral inlet 2036 and an oral outlet 2038. The oral inlet 2036 is where the breathing gas flows into the oral flow passage 2016 of the respiration sensor during expiration, and the oral outlet 2038 is where the ambient air flows into the oral flow passage 2016 of the respiration sensor during inspiration.

In some embodiments, as illustrated in FIG. 17, a cross-sectional area of the oral shroud 2017 forms an hourglass shape. For example, a cross-sectional area of the oral shroud 2017 can taper from the oral inlet 2036 toward the oral thermistor 400-3, positioned between the oral inlet 2036 and the oral outlet 2038, and can taper away from the oral thermistor 400-3 toward the oral outlet 2038. In some embodiments, as illustrated in FIG. 18, the cross-sectional area of the oral shroud 2017 can taper from the oral inlet 2036 toward the oral outlet 2038. The cross-sectional area of the oral shroud 2017 can also taper from the oral outlet 2038 toward the oral inlet 2036.

In some aspects, the oral shroud 2017 can have a cross-sectional profile transverse to a flow through the oral shroud 2017. The cross-sectional profile of oral shroud 2017 can be any regular or irregular shape, such as an oval, circle, square, or rectangle.

FIG. 18 illustrates a detail schematic view of the oral shroud 2017, including an oral flow guide 2034. The oral flow passage 2016 of the oral shroud 2017 collects the breathing gas flow ejected from a patient's mouth. The cross-sectional area of the oral shroud 2017 tapers from the oral inlet 2036 toward the oral thermistor 400-3, and from the oral thermistor 400-3 toward the oral outlet 2038. Alternatively, in some embodiments, the cross-sectional area of the oral shroud 2017 can taper from the oral outlet 2038 to the oral inlet 2036.

The oral flow guide 2034 can direct at least portion of oral respiration flow 2032 moving through the oral flow passage 2016 of the oral shroud 2017. An oral flow guide 2034 is disposed proximate an oral inlet 2036 of the oral shroud 2017 and an oral flow guide 2034 is disposed proximate an oral outlet 2038 of the oral shroud 2017.

During expiration, the oral flow guide 2034 disposed proximate the oral inlet 2036 guides the breathing gas flow through the oral flow passage 2016 and concentrates the breathing gas flow toward the oral thermistor 400-3 while maintaining the breathing gas flow laminar as it passes the oral thermistors 400-3 to minimize turbulent noise. Similarly, during inspiration, the oral flow guide 2034 disposed proximate the oral outlet 2038 guides the ambient air flow through the oral flow passage 2016 of the respiration sensor and concentrates the ambient air flow toward the oral thermistor 400-3 while maintaining the ambient air flow laminar as it passes the oral thermistor 400-3 to minimize turbulent noise.

The oral flow guide 2034 extends from the oral shroud 2017 within the oral flow passage 2016. The oral flow guide 2034 can extend radially inward from an inner surface of the oral shroud 2017. The oral flow guide 2034 can extend across a portion of the oral flow passage 2016, or across the oral flow passage 2016 to engage against an opposite inner surface of the oral shroud 2017. In some embodiments, the oral flow guide 2034 can extend between the oral inlet 2036 and the oral outlet 2038. The oral flow guide 2034 can comprise a surface that is any of a planar, a convex, and a concave surface. In some embodiments, the oral flow guide 2034 is arranged horizontally. In some embodiments, an oral flow guide 2034 is arranged horizontally and another oral flow guide is arranged vertically.

FIG. 19 illustrates a schematic view of the oral flow passage 2016 including an entry angle ε that can create gas flow turbulence 2040. If the entry angle ε is too high, the oral shroud 2017 will create the turbulence 2040 in both directions during inspiration and expiration. FIG. 20 illustrates turbulent noise flow turbulence 2040 during expiration, which is represented by expiration curve 2042 of the measured electrical signal from a thermistor, such as thermistors 400-1, 400-2, 400-3, 500-1, 500-2.

In some embodiments, a cross-sectional area of the oral inlet 2036 mimics a dimension of a patient's open mouth during sleep, but is much less than a fully open mouth and less than a diameter of a patient's forefinger. In some embodiments, the oral inlet 2036 is elliptical in the vertical direction. In such embodiments, the height of the elliptical oral inlet 2036 is approximately 9mm and the width is less than the height, such as approximately 5mm. In some embodiments, the oral outlet 2038 is elliptical. In some embodiments, the oral outlet 2038 is circular. In some embodiments where the both the oral inlet 2036 and the oral outlet 2038 are elliptical, the entry angle ε is relatively small, such that less turbulence is generated, but the gas flow is less concentrated towards the oral thermistor 400-3. In some embodiments, the oral outlet 2038 is approximately 5 mm.

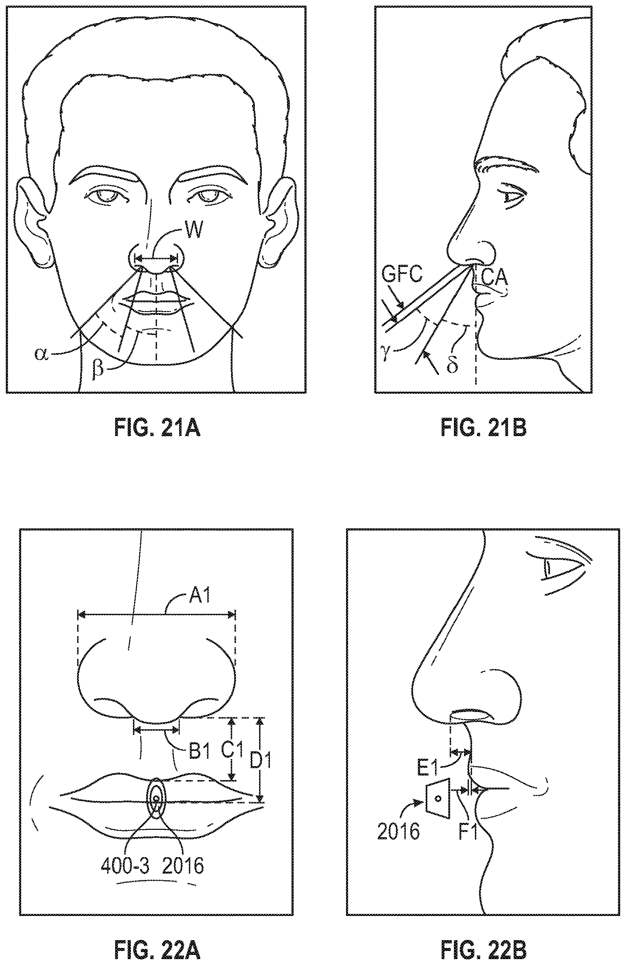

Analysis of entry angle and turbulence generation in the flow cavity, can also be used with reference to the nasal flow passages 301, 2018. FIGS. 21A and 21B illustrate schematic views of possible nasal expiration flow angles, which can be used to determine the potential for turbulence 2040. For example, in a flow path to the side of the nose, an angle α determines a flow width W of the flow path and an angle β determines the direction of the flow path nose. The flow width W is the distance between flow paths from both nostrils. A gas flow column (referred to herein as GFC) is gas flow directed away from the face and the nose. For example, in a flow path directed away from the face and the nose, an angle γ determines a width of the flow path and an angle δ determines the direction of the flow path away from the face and the nose. An area CA defines the cross-sectional surface area of a nostril, which affects the average width of a GFC. In general, a smaller cross-sectional surface area CA of the nostril generates a narrower average width of the GFC. Moreover, turbulence 2040 may be created around the thermistors 400-1, 400-2 by narrow (e.g., low angle α and low cross-sectional surface area CA) breathing GFC that is far to the side of the nose (e.g., high angle β).

### III. Respiration Sensor Size and Adjustability

FIGS. 22A, 22B, 23, and 24, illustrate potential distances or dimensions of a patient's facial features or structures, determination of potential dimensions of the respiration sensor using the measured and average patient facial features, and average measurement results for various patient's facial features or structures.

FIGS. 22A and 22B illustrate potential distances or dimensions of a patient's facial features relative to an oral cavity 2016 having a thermistor 400-3 when the respiration sensor is placed on or attached to the patient. More particularly, the identified dimensions include the patient's nose width A1, isthmus width B1, a distance C1 between the bottom of the nose and the upper lip, a distance D1 between the bottom of the nose and the oral passage (e.g. mouth), a distance E1 between the front edge of the nasal passage and the upper lip, and a lip thickness F1, e.g., the distance the lip protrudes outwardly relative to the philtrum.

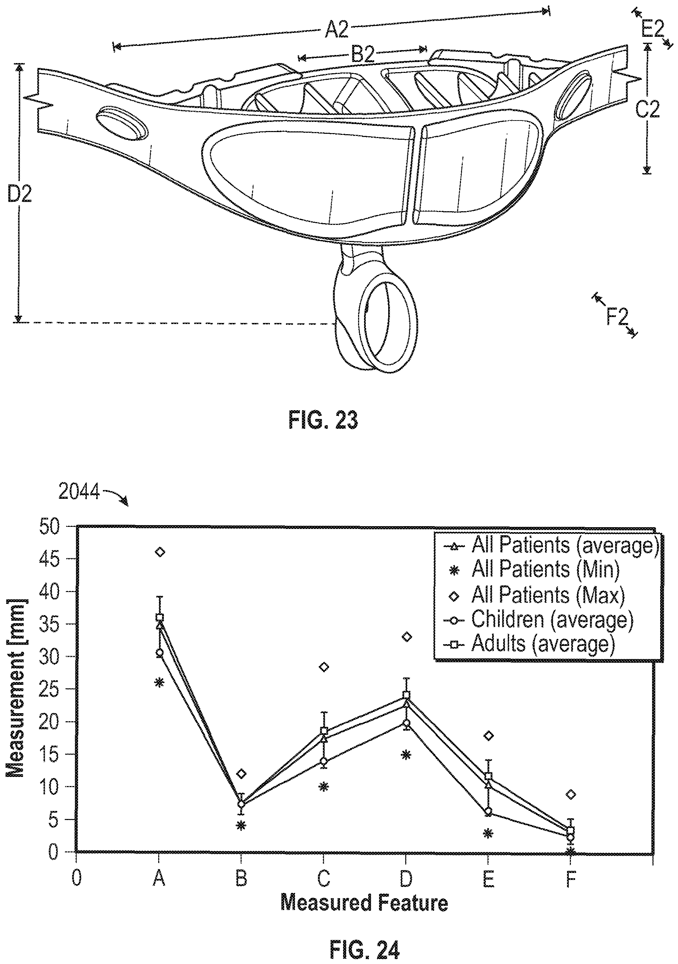

FIG. 23 illustrates a respiration sensor, such as, for example, the respiration sensor 100a, 100b, depicting dimensions of the respiration sensor, which can correspond to analysis of the measured features of the patient as shown in FIGS. 22A and 22B. Accordingly, the measured facial features identified in FIGS. 22A and 22B help facilitate the design dimensions of the respiration sensor 100a, 100b. A2 should be at least A1, but preferably A2 is more than A1 to ensure capturing flow through the patient's nostrils. Similarly, B2 should be no more than B1, but preferably B2 is less than B1 to ensure that B2 does not prevent or disturb flow through the patient's nostrils.

The measured facial features shown in FIGS. 22A and 22B can be used to select design dimensions of the respiration sensor shown in FIG. 23. In some embodiments, measurements of particular patient can be used to select design dimensions for the respiration sensor. In some examples, measurements of a group of patients, such as adults or children, can be used to select design dimensions for an adult respiration sensor or a child respiration sensor.

A measured facial feature can correspond to a design dimension of the respiration sensor. For example: a patient nose width A1 can be used to select the width A2 of the respiration sensor; a patient isthmus width B1 can be used to select the battery frame 2014 width B2; the distance C1 between the bottom of the nose and the upper lip can be used to select a height C2 of the respiration sensor housing 2001; the distance D1 between the bottom of the nose and the oral passage can be used to select a distance D2 between the top of the respiration sensor 100a, 100b, adjacent the nasal inlet 2024 and the oral flow passage 2016, 302; the distance E1 between the front edge of the nasal passage and the upper lip can be used to select a depth of the respiration sensor 100a, 100b; and the lip thickness F1 can be used to select a depth F2 of the oral flow passage 302, 2016.

In some embodiments, the distance C2 of the respiration sensor 100a, 100b is less than 20 mm, but preferably less than 15 mm. In some embodiments, the distance C2 of the respiration sensor 100a, 100b is approximately 10 mm to accommodate different face structures. In some embodiments, width A2 of the respiration sensor 100a, 100b is more than 25 mm, but preferably about 45 mm to adequately capture the gas flow of patients with large width A2. In some embodiments, the distance D2 of the respiration sensor 100a, 100b is more than 5 mm, but preferably more than 10mm. In some embodiments, the distance D2 of the respiration sensor 100a, 100b is more than 15 mm to capture gas flow coming out from the nostrils. In some embodiments, the cross-sectional area of the nasal flow passages 301, 2018 is greater than the cross-sectional area of the nostrils of a patient to capture breathing gas flow. In some embodiments, the battery frame 2014 includes a dimension B2 corresponding to the isthmus width B1 and is preferably less than 10 mm, but more preferably less than 5 mm. In some embodiments, the oral flow passage 2016 is located parallel to the breathing gas flow directed from the mouth of the patient.

FIG. 24 illustrates a graph 2044 of average measurement results for various facial features of a sample of patients including the patient's nose width A1, the isthmus width B1, the distance C1 between the bottom of the nose and the upper lip, the distance D1 between the bottom of the nose and the oral passage (e.g. mouth), the distance E1 between the front edge of the nasal passage and the upper lip, and the patient's lip height F1. The graph 2044 illustrates the measurement results of a group of 45 Caucasian people including women, men, and children between the ages of 0 to 70 years old. The measured values influence the dimensional designs of the respiration sensor 100a, 100b with respect to the nose and the mouth including the size of nasal passages and the location of the oral passage. It should be understood that measurements for patients may also be outside of the scope of the measured feature in this graph.

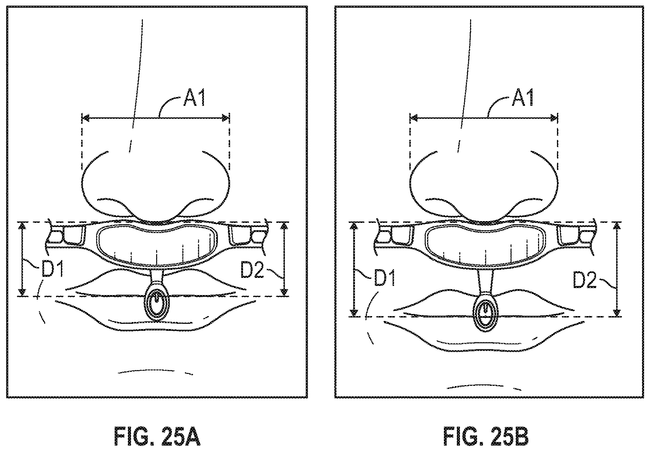

FIG. 25A illustrates a respiration sensor, such as, for example, respiration sensor 100b that includes the distance D2 between the top of the respiration sensor 100b, adjacent the nasal inlet 2024, and the oral flow passage 2016, 302. The distance D2 can be approximately equal to 15 mm for patients with a smaller distance D1. Such a respiration sensor can accommodate patients including a distance D1 in the range of approximately 10 mm to 25 mm. In some embodiments, the distance D1 is between approximately 5 mm to 50 mm.

FIG. 25B illustrates a respiration sensor, such as, for example, the respiration sensor 100b that includes the distance D2 approximately equal to 33 mm for patients with a larger distance D1. Such a respiration sensor can accommodate patients including a distance D1 in the range of approximately 24 mm to 40 mm. In some embodiments, the distance D1 is between approximately 5 mm to 50 mm.

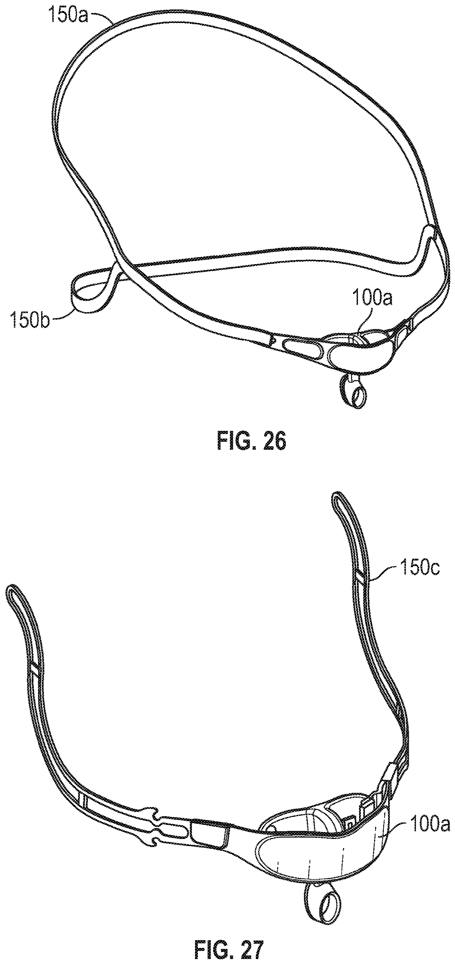

FIGS. 26 and 27 illustrate embodiments of features to attach the respiration sensor 100a to a patient. The features to attach the respiration sensor 100a can include any of a string, strap, or band, which can maintain a position of respiration sensor 100a relative to the patient's physiognomy. It should be understood that any of the features to attach the respiration sensors 100a or 100b can include the features to attach the respiration sensor to a patient.

A strap 150a, shown in FIG. 26, can have ends that are attached to the respiration sensor 100a to form a loop. The strap 150a can have a length such that the respiration sensor 100a is engaged against a patient's face when the device is worn by the patient. In some embodiments, an additional strap 150b extends from any of the strap 150a or the respiration sensor 100a. The additional strap 150b can provide additional support and tension to secure the device with the patient. The strap 150a and additional strap 150b can be configured such that a portion of the strap 150a extends above a patient's ears, and a portion of the additional strap 150b extends below a patient's ears.

FIG. 27 illustrates a respiration sensor 100a having a placement band 150c. In some embodiments, the placement band 150c comprises a semi-rigid framework that is configured to guide straps that overlay the placement band 150c and extend over preferred placement portions of a patient's face. In some embodiments, the placement band 150c comprises a flexible plastic material that is configured to substantially retain its shape during use. The flexible placement band 150c can move, in a first plane, towards or away from a patient's face. The placement band 150c can be moved or biased in the first plane to engage against the patient's face and adapt to the shape of the patient's face. The placement band 150c is less flexible relative to a second plane, transverse to the first plane, thereby preventing or resisting movement of the placement band 150c along the patient's face or twisting of the band 150c.

The placement band 150a, 150c can have a width that is approximately 5mm, but it can be wider or narrower. A wider band can reduce the surface pressure on the face by the band. At least a portion of a surface of the band can be covered with a material that is soft and/or breathable. For example, a surface of the band configured to engage against the face or skin of the patient can comprise a cotton or similar material.

The shape of the band 150c is configured to extend from the respiration sensor 100a, below the cheek bones of the patient. The band 150c can curve from the area below the cheek bones of the patient toward the patient's ears, forming a shape of an S-curve or similar.

The band 150c can be coupled with one or more additional band and/or strap. For example, the band 150c can be coupled to any of straps 150a and 150b. When the straps 150a, 150b pull the band 150c and respiration sensor 100a towards the patient's face, a force vector of the respiration sensor 100a is approximately straight, towards the face or upper lip of the patient. Accordingly, the band 150c can decrease the surface pressure against the patient's isthmus or another portions of the patient's face or lip.

### IV. Respiration Sensor Features for Monitoring and Analysis

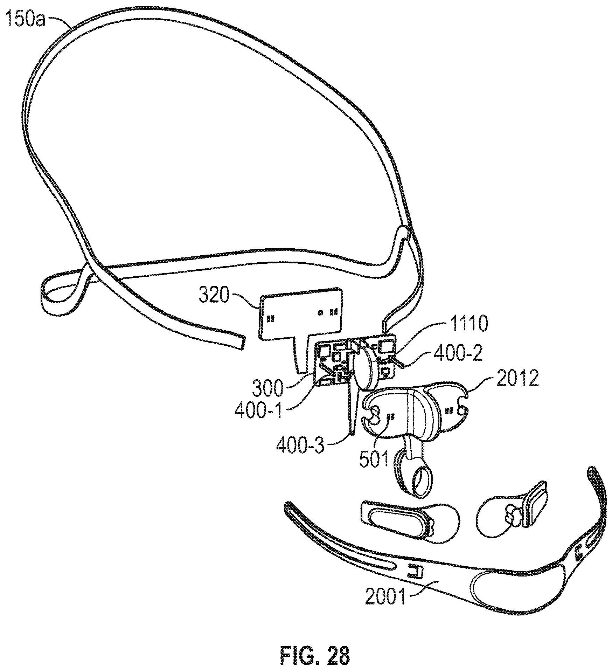

FIG. 28 illustrates an exploded view of the respiration sensor 100a, 100b for example, including a housing 2001, shroud 2012, and electronics board 300, according to some embodiments.

The electronics board 300 includes the electronic components used in the respiration sensor 100a, 100b. The electronics board 300 can include a battery 1110 and sensors, such as a thermistor 400-1, 400-2, 400-3, and a capacitive plate. In some embodiments, the electronics board 300 is made of, for example, glass-reinforced epoxy laminate material (e.g., FR4 substrate) containing automatic machine placed components, commonly used in automated mass series production to make the construction low cost. The electronics board 300 can be coupled to a base plate or frame 320. In some embodiments, the frame 320 includes plastics, which contains electrically conductive areas or conductors.

In some embodiments of the present disclosure, the battery 1110 can be a disposable or rechargeable battery. In some embodiments, the respiration sensor 100a, 100b is configured to be powered by solar energy. For example, the respiration sensor 100a, 100b can include a solar panel which can be coupled to a battery.

The shroud 2012 defines at least a portion of the nasal flow passages and the oral flow passage of the sensor. In some embodiments, the electronics board 300 is positioned between the frame 320 and the shroud 2012. Any of the frame 320 and the shroud 2012 can include a cavity to protect the electronics board 300 when the respiration sensor 100a, 100b is assembled. The frame 320 and/or the shroud 2012 can be made of elastic silicone, plastics, or similar material.

In some embodiments, an ambient air thermistor is positioned further away from the breathing gas flow otherwise interfering ambient air measurement. In aspects of the present disclosure, the shroud 2012 can include a perforation 501 that enables ambient air to be in touch with the ambient air thermistor through the shroud 2012 to get fast response time, but also to protect ambient air thermistor for example from touching with a finger or any unwanted air flow, such as air conditioning.

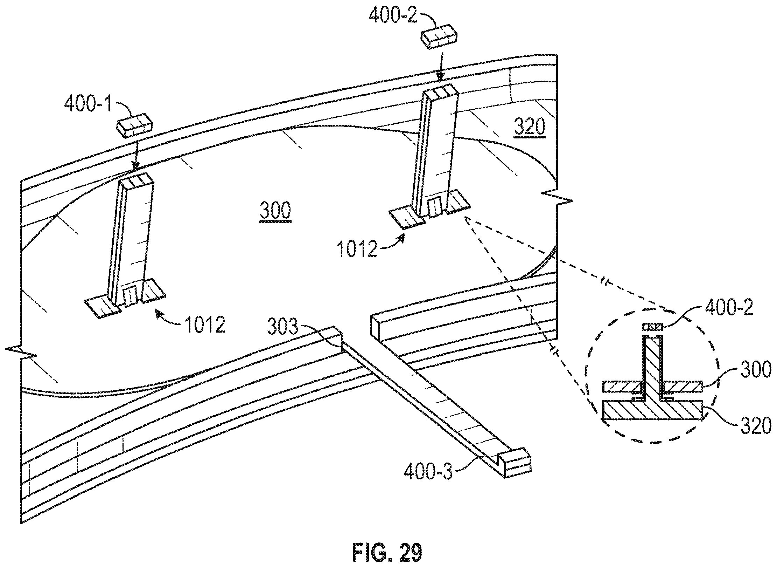

Referring to FIG. 29, a portion of the frame 320 can form a support structure for the thermistors 400-1, 400-2. The electronics board 300 may include two perforations that enable two poles of the frame 320 to pierce through the electronics board 300 to form the thermistor support structure. The poles locate and keep the board in place with a mechanical locking mechanism. No screws or similar are needed. The poles also contain electrical contacts on the tip of the poles where thermistors 400-1, 400-2, which are sensitive to nasal breathing gas flow, are coupled. Electrically conductive connections 1012 on the side surfaces of poles further connect thermistors 400-1, 400-1 to the electronics board 300 via electrical contacts on the top surface of the electronics board 300 next to the poles. When the frame 320 is placed under the electronics board 300, electrical contacts on the top surface of the frame 320 connect with adjacent electrical contacts on the bottom surface of electronics board 300. Electrically conductive glue can be used to ensure electrical contact. In some embodiments, a thermistor 400-3 sensitive to breathing gas flow through the mouth is located to the tip of the electronics board 300. A bottom side of frame 320, adjacent to electronics board 300 contains an inset 303 to enable thermistor 400-3 to locate into the middle of the flow cavity.

Electrical signals from thermistors 400-1, 400-2, 400-3 proportional to corresponding ambient, skin, nasal or oral temperature changes are conducted through the electrically conductive connections 1012 and conductors to central processing unit on the electronics board. The central processing unit can convert the analog data into digital form, process and transmit the data wirelessly, for example, via an RF transmitter, to a host where the data can be shown or displayed to a caregiver in a suitable form of numbers and/or waveforms.

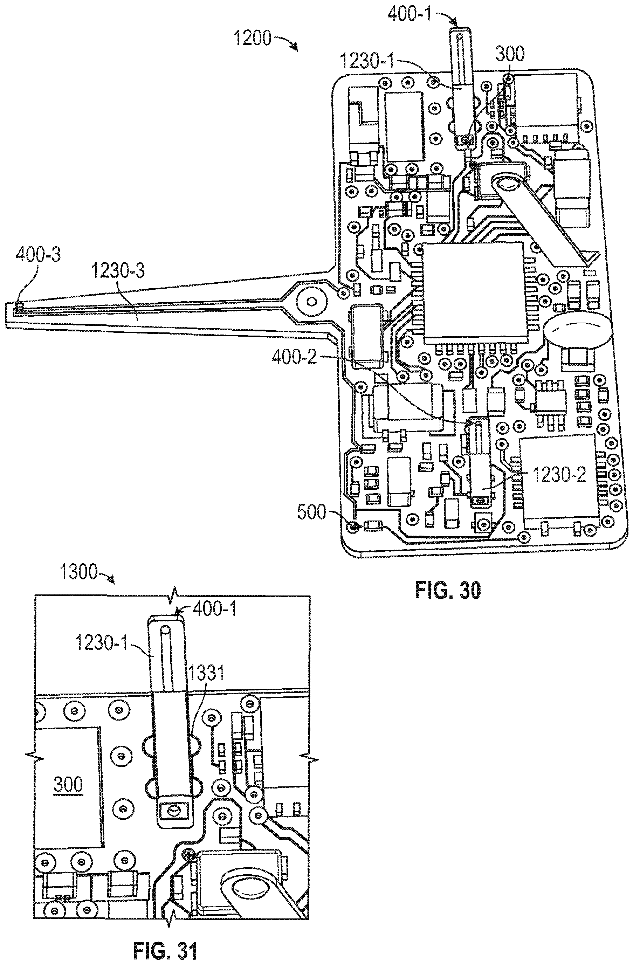

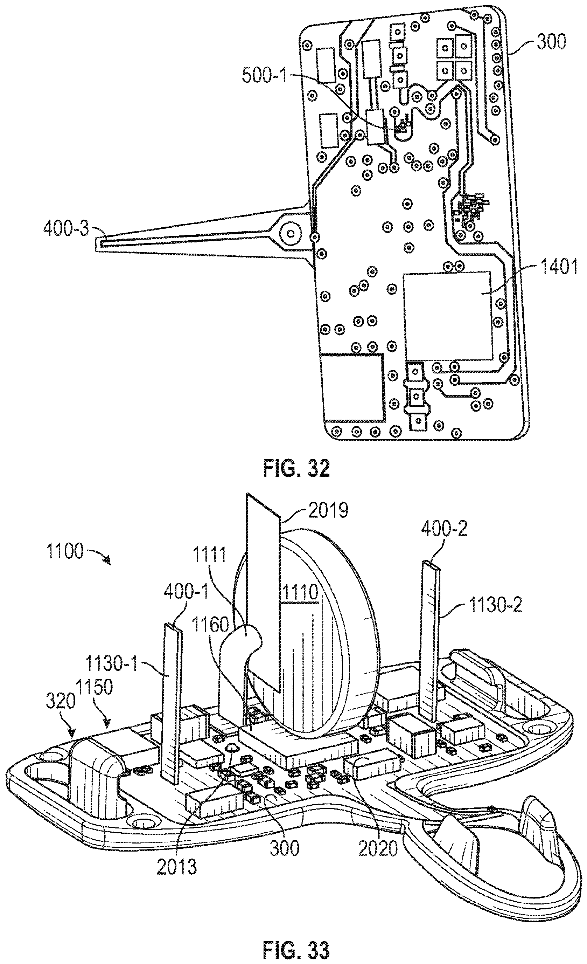

FIG. 30 illustrates a detailed view of an electronics assembly 1200, for example, any respiration sensor 100a, 100b which can include two nasal flow thermistors 400-1, 400-2 and one oral flow thermistor 400-3, according to some embodiments. Thermistors 400-1 and 400-2 are configured to measure breathing from nostrils. Thermistor 400-3 may be configured to measure breathing from the mouth. A thermistor 500-1 (see FIG. 32) may also be included in electronics assembly 1200 to measure ambient temperature.

Support structures 1230-1, 1230-2, 1230-3 contain electrical wires on both sides of a strip between electrical connections at both ends of the strips. The support structures can include first and second support structures 1230-1, 1230-2, which can support the nasal flow thermistors 400-1, 400-2. Additionally, a third support structure 1230-3 can support the oral flow thermistor 400-3. In some embodiments, support structures 1230-1, 1230-2, 1230-3 may include an electrically and thermally insulating material (e.g., FR4 substrate). Thermistors 400-1, 400-2, 400-3 can be soldered to electrical connections in the first end of the strips. Second ends of strips are placed into small holes in electronics board 300 and soldered to form electrical connections on the sides of the strip to corresponding electrical contacts on the board to electrically connect thermistors 400-1, 400-2, 400-3 to sensor electronics in the plane of the electronics board.

The cross-sectional areas of copper or similar traces within support structures 1230-1, 1230-2, 1230-3 are reduced to minimize thermal flow through the electrical conductors from the plane of board to thermistors 400-1, 400-2, 400-3. To minimize the thermal mass of the thermistors 400-1, 400-2, 400-3, the support structures 1230-1, 1230-2, 1230-3 can be formed from a thermally non-conductive or insulating material. These optimizations make thermistors 400-1, 400-2, 400-3 as sensitive as possible to thermal changes caused by the breathing gas flowing past the thermistor during expiration or ambient gas flowing past the thermistor during inspiration.

FIG. 31 illustrates a partial view 1300 of an electronics board 300 in, for example, any respiration sensor 100a, 100b including details of a nasal flow thermistor 400-1, according to some embodiments. Support structure 1230-1 may include an FR4 substrate strip with thermistor 400-1 placed on the tip of the strip. At the bottom of support structure 1230-1, a soldered contact provides electrical contacts to thermistor 400-1 on both sides of support structure 1230-1 (e.g., +/- terminals).

In some embodiments, the support structures 1230-1, 1230-2, can have a proximal portion coupled to the electronics board 300 and a distal portion transverse to a plane defined by the top of the electronics board 300. When the electronics board is positioned within the housing, the distal portion of the support structures 1230-1, 1230-2 can extend into respective nasal flow passages. In some embodiments, the support structure 1230-3 can have a proximal portion coupled to the electronics board 300 and a distal portion that is normal with or substantially parallel to a plane defined by the top of the electronics board 300.

FIG. 32 illustrates a detailed view of a bottom portion of an electronics board 300 of a respiration sensor 100a, 100b including a thermistor 500-1 to measure skin temperature, and a capacitive plate or sensor 1401 to measure sensor location in the upper lip, according to some embodiments. A respiration sensor including electronics board may be turned on/off based on the signal. Additionally, in some embodiments, the electronics board 300 includes an accelerometer 1150.

FIG. 33 illustrates a detailed view of an electronics board 300 coupled with a frame 320 and a battery 1110, according to some embodiments. Support structures 1130-1 and 1130-2 extend away from the electronics board, and include thermistors 400-1 and 400-2, respectively. A thermistor 500-1 sensitive to skin temperature may be located on the bottom side of the board as close to skin as possible. In some embodiments, the thermistor is placed close to one of the two ridges above the upper lip to ensure closest distance to skin. The frame 320 most advantageously contains a perforation adjacent to thermistor that enables better thermal contact to upper lip skin. Perforation can also be filled with thermally conductive material to increase conductivity to skin.

The electronics board 300 includes a battery contact tab 1111 that extends toward the battery 1110. A portion of the spacer 2019 is positioned between the battery contact tab 1111 and the battery 1110 such that the contact tab 1111 is spaced apart from the battery 1110. When the spacer 2019 is coupled with the respiration sensor 100b, the battery 1110 the battery does not provide power to the respiration sensor 100b.

In some embodiments, the board 300 includes an LED 2013, which can be visible from an outer surface of the respiration sensor 100b when the respiration sensor 100b is assembled. In some embodiments, the board 300 includes a microphone 2020. The microphone 2020 can detect ambient sounds or a patient speaking. The sound detected by the microphone 2020 can be used to during processing of signals. For example, the sound detected by the microphone 2020 can filtered out to reduce or remove noise in the signals from the other sensors.

In some embodiments, any respiration sensor 100a, 100b is an affordable, disposable, wireless sensor configured to detect breath flow in real time. Accordingly, the sensor 100a, 100b includes a battery 1110, which may provide several days (e.g., five days, or more) of continuous, real time, fast response operation with a high signal quality. In some embodiments, the respiration sensor 100a, 100b is configured to measure a respiration rate (RR) and magnitude, and to provide real time respiration waveforms, in digital and/or analog form. Furthermore, a processor circuit in the respiration sensor may be configured to determine trends and projections based on the real-time data (e.g., via moving averages, Kalman filtering, and the like). The respiration sensor 100a, 100b may also provide skin temperature, body position, movement, fall detection (e.g., through an accelerometer 1150), sensor placement, and the like.

### V. Processing of Readings for Indications

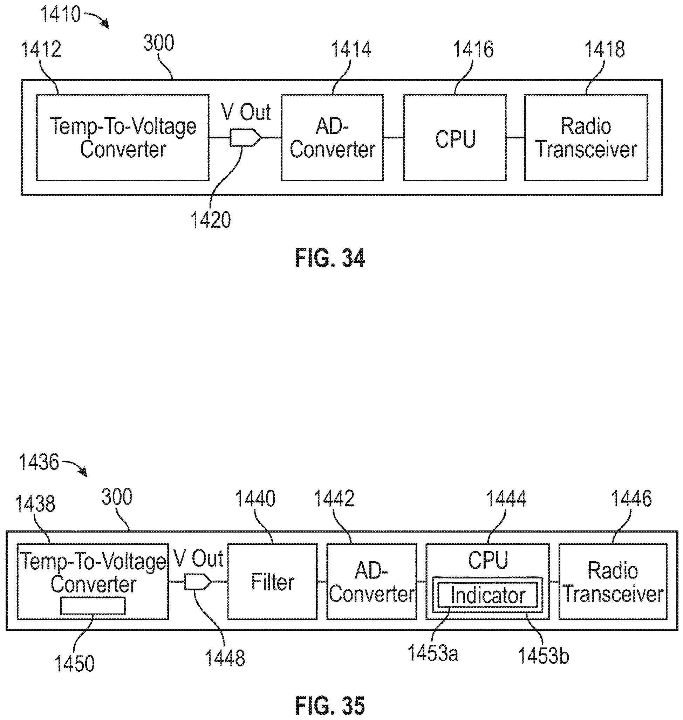

FIG. 34 illustrates a block diagram 1410 of components, which are utilized on the electronics board 300 of the respiration sensor 100a, 100b according to some embodiments. In such embodiments, the electronics board 300 includes a temperature-to-voltage converter 1412, an analog-to-digital (AD) converter 1414, a central processing unit (CPU) 1416, and a communications module or radio transceiver 1418 for providing a two-way data communication coupling to a network link that is connected to a local network. Such communication may occur, for example, through a radio-frequency transceiver. In addition, short-range communication may occur, such as using a Bluetooth, Wi-Fi, or other such transceiver. In some embodiments, the CPU 1416 includes the Bluetooth low energy processor 1160 (shown in FIG. 33).

In some embodiments, the temperature-to-voltage converter 1412 includes any of the thermistor 500-1, the thermistor 500-2, the thermistor 400-1, the thermistor 400-2, and the thermistor 400-3. In some embodiments, any of the thermistors 400-1, 400-2, 400-3, 500-1, 500-2 are negative temperature coefficient (NTC) type thermistors, such that the thermistor's electrical resistance decreases when the temperature increases. In some other embodiments, any of the thermistors are positive temperature coefficient (PTC) type thermistors, such that the thermistor's electrical resistance increases when the temperature increases. The respiration sensor 100a, 100b can include any combination of NTC type thermistors and PTC type thermistors. The temperature-to-voltage converter 1412 converts or transforms the temperature resistance value detected at any of the thermistors to a voltage at Vout 1420. The AD converter 1414 then converts the Vout 1420 into digital form, which is received by the CPU 1416 for further processing and calculations. In some embodiments, the CPU 1416 can transmit the digital signal to the host monitor or other client device. The CPU 1416 can transmit the digital signal via the Bluetooth low energy processor 1160. In some other embodiments, the CPU 1416 transmits the digital signal to the communications module or radio transceiver 1418 for wireless transmission to the host monitor or other client device.

In addition to the respiration sensor 100a, 100b measuring or detecting temperature differences between inspiratory and expiratory gas flows via the thermistors 400-1, 400-2, 400-3, the respiration sensor 100a, 100b also measures or detects ambient air temperature via the thermistor 500-1 and conductive temperature from the patient's skin via the thermistor 500-2.

In some instances, the thermistor 500-1 and the thermistor 500-2 include a wide operating temperature range and can be adjusted to include a lowest and a highest temperature of operating range. The respiration sensor 100a, 100b is configured to measure or detect the electrical signal voltages proportional to the ambient air temperature via the thermistor 500-1 and the skin temperature via the thermistor 500-2 and compensate the signal offset, gain, and the peak to peak amplitude errors from the inspiratory and expiratory gas flow signal amplitude.

In some embodiments, any of the thermistors 400-1, 400-2, 400-3, 500-1, 500-2 can measure any of an inspiratory gas flow, an expiratory gas flow, an ambient air temperature, and a conductive temperature. For example, when the respiration sensor 100a, 100b is turned on, but is not yet placed on or attached to the patient's face, the thermistor 400-1, 400-2, 400-3, 500-1, 500-2 detect ambient air temperature. When the respiration sensor 100a, 100b is placed on or attached to the patient's face, the thermistor 500-2 begins detecting the temperature of skin on the patient's upper lip. Meanwhile, the thermistor 500-1 remains detecting the ambient air temperature and the thermistors 400-1, 400-2, 400-3 begin detecting the temperature differences between the inspiratory and the expiratory gas flows (e.g., inspired ambient air and expired warm gas coming out from the lungs).

During normal, stable ambient conditions, after the respiration sensor 100a, 100b is placed on or attached to the patient's face, the electrical voltage signals from the thermistor 500-2 (e.g., detecting ambient air temperature) are stable and change slowly, whereas the electrical voltage signal from at least one of the thermistors 400-1, 400-2, 400-3 changes its amplitude relatively faster. In some embodiments where the thermistors are NTC type and the temperature-to-voltage converter 1412 includes negative feedback amplifiers, the electrical voltage signal changes between maximum voltage proportional to ambient air temperature and minimum voltage proportional to temperature of exhaled warm, moister gas coming out of the patient's lungs.

In other embodiments where the thermistors 400-1, 400-2, 400-3, 500-1, 500-2 are PTC type and the temperature-to-voltage converter 1412 include positive feedback amplifiers, the electrical voltage signal changes between maximum voltage proportional to temperature of exhaled warm, moister gas coming out of the patient's lungs and minimum voltage proportional to ambient air temperature. In both NTC type and PTC type scenarios, the frequency of electrical signal may vary between 0 to 3 Hz (0-180 RR/min) depending on how fast the patient is inhaling and exhaling. Smaller patients tend to breathe relatively faster than relatively larger patients, such as adults.

FIG. 35 illustrates a block diagram 1436 of components, which are utilized on the electronics board 300 of the respiration sensor 100a, 100b according to some embodiments. In such embodiments, the electronics board 300 includes a temperature-to-voltage converter 1438, a filter 1440, an analog-to-digital (AD) converter 1442, a central processing unit (CPU) 1444, and a communications module 1446 for providing a two-way data communication coupling to a network link that is connected to a local network. Such communication may occur, for example, through a radio-frequency transceiver. In addition, short-range communication may occur, such as using a Bluetooth, Wi-Fi, or other such transceiver. In some embodiments, the CPU 1444 includes the Bluetooth low energy processor 1160 (shown in FIG. 33).

In some embodiments, the temperature-to-voltage converter 1438 includes any of the thermistors 400-1, 400-2, 400-3, 500-1, 500-2. In some embodiments, any of the thermistors are negative temperature coefficient (NTC) type thermistors, such that the thermistor's electrical resistance decreases when the temperature increases. In other embodiments, any of the thermistors are positive temperature coefficient (PTC) type thermistors, such that the thermistor's electrical resistance increases when the temperature increases. The respiration sensor 100a, 100b can include any combination of NTC type thermistors and PTC type thermistors. The temperature-to-voltage converter 1438 converts or transforms the temperature resistance value detected at one of the thermistors 400-1, 400-2, 400-3, 500-1, 500-2 to a voltage at Vout 1448. In some embodiments, the temperature-to-voltage converter 1438 also includes an amplifier 1451, which increases the voltage at Vout 1448 for increased accuracy and resolution of the breathing gas flow signal.

The filter 1440 eliminates or subtracts any of the ambient air and the conducting skin temperature change from the breathing gas flow signal. The AD converter 1442 then converts the signal from the filter 1440 into digital form, which is received by the CPU 1444 for further processing and calculations. In some embodiments, the CPU 1444 can transmit the digital signal to the host monitor or other client device via the Bluetooth low energy processor 1160. In some other embodiments, the CPU 1444 transmits the digital signal to the communications module 1446 for wireless transmission to the host monitor or other client device. In some embodiments, the filter 1440 is configured to subtract the electrical signal detected by the thermistor 500-2 from the electrical signal detected by the thermistor 500-1. In some embodiments, the filter 1440 is configured to subtract the electrical signal detected by the thermistor 500-2 and the electrical signal detected by any of the nasal thermistor 400-1, the nasal thermistor 400-2, and the oral thermistor 400-3 from the electrical signal detected by the thermistor 500-1.

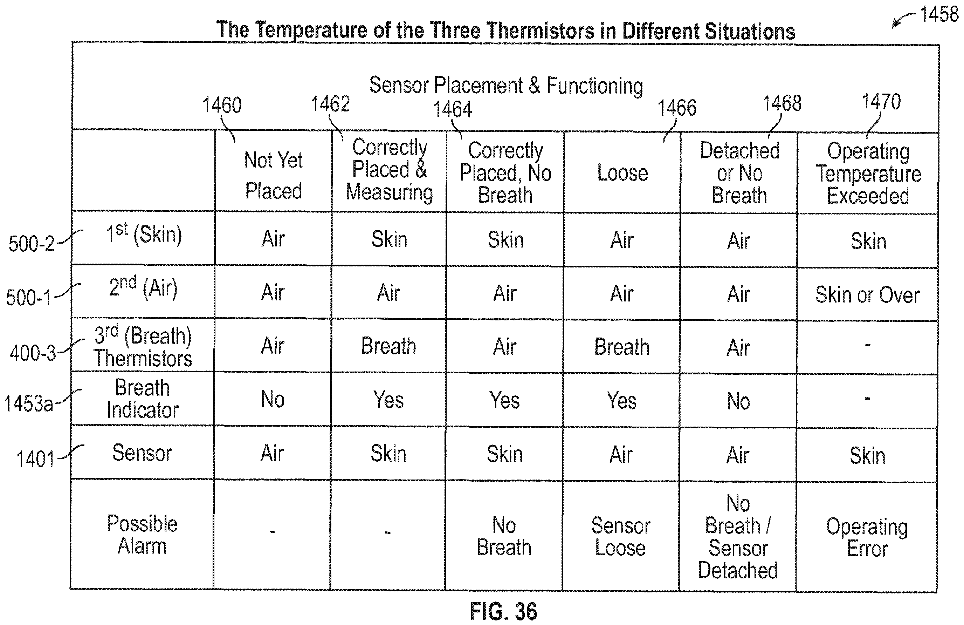

FIG. 36 illustrates a respiration sensor detection state table 1458 for determining the respiration sensor placement and function. For example, an operation logic is derived from the electrical signals from the thermistor 500-1, the thermistor 500-2, and the thermistor 400-1, 400-2, 400-3 to detect different states of the respiration sensor 100a, 100b. The different states of the respiration sensor 100a, 100b are utilized to identify sensor placement with respect to the patient and function of the sensor for monitoring and notifying of these states. The respiration sensor 100a, 100b is capable of identifying, for example, early signs of respiratory depression, spasms, obstructions, and other symptoms, and notifying of these identifications. In addition to notifying of such identifications, the respiration sensor 100a, 100b is also capable of notifying when an improper placement of the sensor is identified or detected to alert a caregiver to check on the patient and make sure that the sensor is not obstructing the patient's airways or otherwise disturbing the patient.

The respiration sensor 100a, 100b includes various detection states including, but not limited to: a not-yet-placed state (Not Yet Placed state 1460); a correctly-placed and measuring state (Correctly Placed & Measuring state 1462); a correctly-placed, but no breath state (Correctly Placed, No Breath state 1464); a loose device state (Loose state 1466); a detached or no breath state (Detached or No Breath state 1468), and an operating-temperature exceeded state (Operating Temperature Exceeded state 1470).

In the Not Yet Placed state 1460, the respiration sensor 100a, 100b is not yet placed on the patient. For example, when the respiration sensor 100a, 100b is turned on, but not yet placed on or attached to the upper lip of the patient, the thermistor 500-2, the thermistor 500-1, and the thermistor 400-3 all detect a similar signal corresponding to temperature proportional to ambient temperature and the breath indicator 1453a (shown in FIG. 35) detects no breaths. Under these detected conditions, the respiration sensor 100a, 100b determines that it is in the Not Yet Placed state 1460 and will not transmit an alert notification.

After the respiration sensor 100a, 100b is placed on or attached to the upper lip of the patient, the thermistor 500-2 detects and adapts to a temperature close to skin temperature of the upper lip while the thermistor 500-1 remains detecting the ambient air temperature. In some embodiments, the temperature offset error in the thermistor 500-2, which is caused by, for example, a mustache, may be ignored since the temperature detection is enough to monitor the temperature change during the time it takes to detect or determine whether the sensor is in proper placement or not (e.g., not the absolute value). When the location of the sensor between the nasal and the oral passages of the patient is proper and the patient is breathing the thermistor 400-3 start to adapt to and detect the temperature of the sequentially changing gas flow (e.g., Breath). At this point, the breath indicator 1453a determines that the thermistor 400-3 detected a breath. Under these conditions, the respiration sensor 100a, 100b determines that it is in the Correctly Placed & Measuring state 1462 and will not transmit an alert notification.

The respiration sensor 100a, 100b determines that it is in the Correctly Placed, No Breath state 1464 when the thermistor 500-2 remains detecting and adapting to the skin temperature and the thermistor 500-1 remains detecting the ambient air temperature, but the thermistor 400-3 no longer sufficiently adapts or detects the gas flow temperature (e.g., detects ambient air temperature instead) even though the breath indicator 1453a detects breaths. In the Correctly Placed, No Breath state 1464, the location of the respiration sensor 100a, 100b between the nasal and/or oral cavities may be unsatisfactory and the gas flow through the sensor cavities may be insufficient and the respiration sensor 100a, 100b will transmit an alert notification indicating that "No Breath" is detected. It is also possible that, in the Correctly Placed, No Breath state 1464, the patient is not breathing sufficiently enough and needs immediate attention from clinical personnel.

The respiration sensor 100a, 100b determines that it is in the Loose state 1466 when the thermistor 500-2 does not detect the skin temperature and detects, instead, a similar value as the thermistor 500-1 (e.g., ambient air temperature) while the thermistor 500-1 remains detecting the ambient air temperature, the thermistor 400-3 detects the gas flow temperature, and the breath indicator 1453a detects breaths. In the Loose state 1466, the respiration sensor 100a, 100b may be positioned askew with respect to the upper lip of the patient, such that breathing gas flow is not properly detected or monitored, and the respiration sensor 100a, 100b will transmit an alert notification indicating that a "Loose Sensor" is detected so that care personnel may adjust the respiration sensor 100a, 100b with respect to the patient's upper lip.

The respiration sensor 100a, 100b determines that it is in the Detached or No Breath state 1468 when the thermistor 500-2, the thermistor 500-1, and the thermistor 400-3 all detect ambient air temperature and the breath indicator 1453a detects no breaths. In the Detached or No Breath state 1468, the respiration sensor 100a, 100b is detached from the patient and it will transmit an alert notification indicating "Sensor Detached." In some embodiments, in the Detached or No Breath state 1468, in addition to or alternatively, the respiration sensor 100a, 100b will transmit an alert notification indicating that "No Breath" is detected.

The respiration sensor 100a, 100b determines that it is in the Operating Temperature Exceeded state 1470 when temperature detected by the thermistor 500-1 equals or exceeds the temperature detected by the thermistor 500-2. This means that the ambient temperature is too close to the breathing gas temperature to give sufficient differential temperature readings, which is proportional to the respiration signal amplitude. Such a situation may occur when the patient is lying face downward against a surface (e.g., bed or pillow). In the Operating Temperature Exceeded state 1470, the respiration sensor 100a, 100b will transmit an alert notification indicating an "Operating Error."

In some embodiments, signals from the nasal thermistors 400-1, 400-2 are compared to determine a state of the patient or the respiration sensor 100a, 100b. The signal from the nasal thermistors 400-1, 400-2 can be compared relative to each other to determine if the respiration sensor 100a, 100b is correctly placed on the patient. For example, a normal signal from thermistor 400-1 or 400-2, and a low or non-existent signal from the other of thermistor 400-1 or 400-2, can indicate that the respiration sensor 100a, 100b is not positioned correctly relative to the patient's nostrils.

The capacitive sensor 1401 can also be used to activate and/or turn on the respiration sensor 100b. In some embodiments, the processor can be set into a low power or sleep mode when the respiration sensor 100b is in storage or not in use. When in the sleep mode, the respiration sensor 100b can process a measured value from the capacitive sensor 1401 and compare the measured value to a previous value stored into the memory. The previous value stored into the memory can correspond to a respiration sensor 100b that is not engaged against a patient's face. When the respiration sensor 100b is placed on a patient's upper lip, the capacitive value measured by the capacitive sensor 1401 can change. The change of capacitive value can be caused by the capacitive sensor 1401 engaged against the patient's lip or tissue, which can have a different permeability relative to another material such as the capacitive sensor 1401 packaging or ambient air.

When a change in measured value from the capacitive sensor 1401 is detected, the processor can change the sensor from the low power or sleep mode to a normal operating mode., In some embodiments, the processor can activate other electrical circuits on the electronics board when a change in measured value from the capacitive sensor 1401 is detected. In some embodiments, when the respiration sensor 100b is separated from the face of a patient, and a measured value from the capacitive sensor 1401 corresponds to a respiration sensor that is not engaged against a patient's face, the respiration sensor 100b can turn off. In some embodiments, the respiration sensor 100b can turn off when the capacitive sensor 1401 detects a change back to the permeability of, for example, air and/or no breathes are detected. In some embodiments, the respiration sensor 100b can wait for a predetermined safety time limit, e.g., 5 minutes, and then turn off or enter a low power mode.

In some embodiments, the respiration sensor 100a, 100b can begin measurement automatically when the processor counts one or more breaths from any of the nasal and oral thermistors. For example, measurement can start automatically when the processor counts three different successful breaths from the nasal and/or oral thermistors.

To determine the respiration sensor placement and function, the electronics board 300 can include, for example, any of a Bluetooth low energy processor 1160, the temperature-to-voltage converter 1438, the filter 1440, the AD converter 1442, the CPU 1444, the communications module 1446, and the breath indicator 1453a stored in the memory 1453b.

The amplitude of the alternating electrical voltage signal from the thermistors 400-1, 400-2, 400-3, 500-1, 500-2 can be converted proportional to a real temperature, for example into degrees of Celsius. In principle, when the patient breathes normally, the minimum amplitude of electrical signal from the NTC type thermistors is proportional to the maximum temperature of exhaled air and the maximum amplitude of electrical signal from the NTC type thermistors is proportional to the minimum temperature of inhaled ambient air. For the PTC type thermistors, the maximum amplitude of electrical signal is proportional to the maximum temperature of exhaled air and the minimum amplitude of electrical signal is proportional to the minimum temperature of inhaled ambient air. The conversion from electrical voltage signal to temperature is negative with NTC type thermistor, whereas the conversion from electrical voltage signal to temperature it is positive with PTC type thermistor. Accordingly, both NTC and PTC type thermistors can provide the same temperature value.

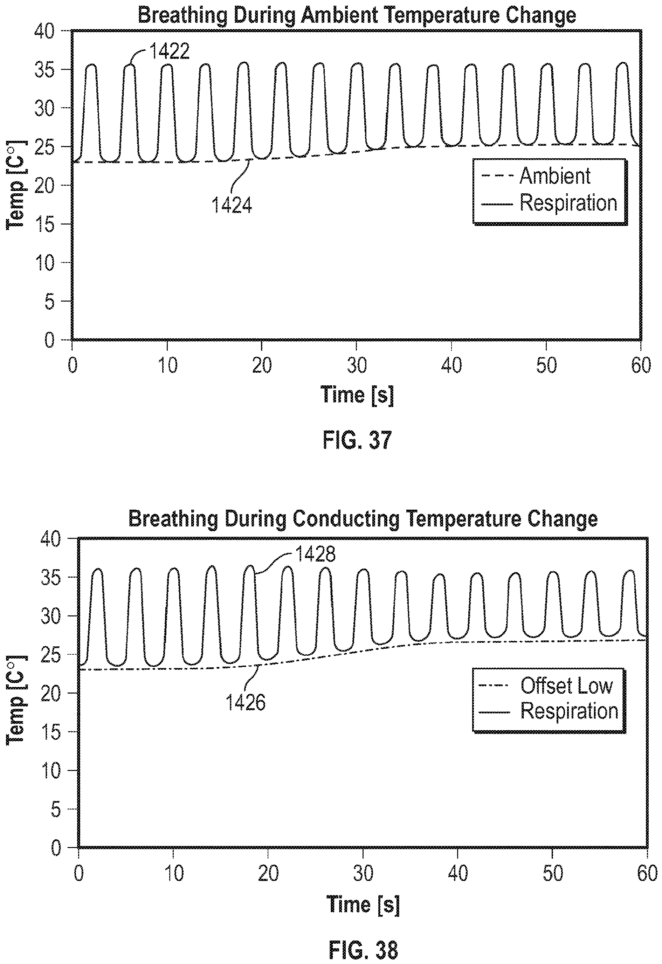

FIG. 37 illustrates the temperature of breathing (respiratory flows) during changes in ambient air temperature. The amplitude of the alternating breathing signal 1422 indicates a temperature difference between inhaled ambient air and exhaled gas from the lungs in degrees of Celsius [C°], and can be proportional to a strength of breathing or volume and flow of breathing. The peak-to-peak amplitude of alternating breathing signal 1422, presented in degrees of Celsius [C°], depends mostly on the flow rate of gas and ambient air temperature 1424. As can been seen in FIG. 37 the peak-to-peak amplitude of the breathing signal 1422 decreases when the ambient air temperature 1424 increases.

In some instances, if exhaled breathing gas flow and volume decreases, the measured signal amplitude decreases proportionally. Due to lower gas volume there is less thermal energy, and due to lower gas flow speed, exhaled gas has more time to release thermal energy to surrounding air and sensor housing (e.g., housing 2001) before reaching the thermistor 400-1, 400-2, 400-3. Additionally, the exhaled gas can have less thermal energy to warm up the thermistor 400-1, 400-2, 400-3.

In some instances, if ambient air temperature decreases, the exhaled gas releases even more energy due to higher energy difference between two gas mediums. On the other hand, the maximum breathing signal is proportional to ambient temperature, and is sensitive to ambient air temperature changes, thus the peak-to-peak signal amplitude proportional to sequentially changing inhaled and exhaled gas is also dependent on ambient air temperature. In some instances, if ambient air temperature increases, the breathing signal amplitude between inspirations and expirations decreases and, vice versa, the breathing signal amplitude between inspirations and expirations increase when ambient air temperature decreases.

In some embodiments, energy in the form of heat from a patient's upper lip can be conducted through the respiration sensor housing to thermistors. In some instances, energy directed from or toward a gas flowing through the respiration sensor 100a, 100b can cause a similar effect as ambient air change. FIG. 38 illustrates breathing (respiratory flows) during a change in thermal energy conducting temperature, for example when the respiration sensor 100a, 100b is coupled to a patient's face. The sensor housing (e.g., housing 2001) that guides gas flow through the respiration sensor 100a, 100b is preferably made of plastic, silicon, or similar material with low thermal coefficient to minimize its ability to absorb, store, and conduct thermal energy. However, the housing may conduct some thermal energy from patient's upper lip and elevate the sensor temperature, similar to ambient temperature change. The change in temperate generates a small offset, represented by offset curves 1426, to the temperature signal 1428 proportional to inhaled ambient air temperature decreasing the breathing signal peak-to-peak amplitude. The thermistor 400-1, 400-2, 400-3 senses when thermal energy, stored during expiration, is released during inspiration, and senses when thermal energy is released during the expiration phase, thus decreasing the breathing signal peak-to-peak amplitude between inspired and expired phases. This offset, represented by the offset curves 1426, can dependent on any of the ambient air temperature and the thermal coefficient of the sensor housing's material, which is a constant based on laboratory measurement and can be taken into account. The thermal energy conducting from a patient's upper lip through the sensor housing is strongly dependent on the thermal connection between the respiration sensor 100a, 100b and the patient's face, which in turn is proportional to temperature and the electrical signal from the thermistor 500-2.

When the respiration sensor has been placed on patient's face, each of the inlets to the nasal flow passage cavities can be separated from the corresponding nasal outlet of the patient, and the inlet to the oral flow passage cavity can be separated from the corresponding oral outlet of the patient (i.e., mouth). When the patient breathes, warm and moist breathing gas flows through any of the nasal and oral flow passages. Warm and moister exhaled breathing gas releases thermal energy into the ambient air if the ambient air temperature is lower than the exhaled air temperature. The temperature of exhaled air decreases as shown in FIG. 38 represented by the offset curves 1426, which decreases the breathing signal amplitude. To get maximal breathing signal amplitude during exhalation, the sensor housing or respiration sensor cavities are positioned as close as possible to patient's nasal and oral passage cavities.

It can be important to have accurate breathing signal peak to peak amplitude proportional to patient's actual inspired and expired breathing efforts at any time and during any condition to be able to detect situations, such as, for example, opiates deteriorating patient's breathing, obstructions, bronchospasms, etc. Changes in ambient air temperature and in conducting thermal energy may cause a decrease in the peak-to-peak signal amplitude, which resembles a similar decrease, for example, as when opiates deteriorate the patient's breathing. In order to correctly identify or detect the cause of the decrease and to avoid a misidentification, such error signals can be compensated and eliminated to prevent any false notifications of these error signals. Ambient air temperature changes that decrease the breathing gas signal can be compensated and eliminated based on the temperature signal proportional to the thermistor 500-1 sensitive to the ambient temperature. Compensation to the breathing gas signal is inversely proportional to increases in the ambient air temperature, thus if the ambient air temperature increases, then the gain of the breathing gas signal is increased, and vice versa. Similarly, changes in skin temperature, which is proportional to the conducting temperature through the sensor housing 2001, also decrease the breathing gas signal and is compensated and eliminated based on the temperature signal proportional to the thermistor 500-2 sensitive to the skin temperature. Compensation to the breathing gas signal is inversely proportional to increases in the skin temperature, thus if the skin temperature increases, then the gain of the breathing gas signal is increased, and vice versa.

In some embodiments, thermal transients can be eliminated and signal amplitude relative to ambient and thermal energy conducting temperatures can be compensated to produce a respiratory flow signal. For example, after removing the thermal effects as discussed above with reference to FIG. 35, the breathing gas flow signal may be displayed at the host monitor or other client device. The accuracy and resolution of the breathing gas flow signal is enhanced due to the elimination of the thermal transients and compensating the signal amplitude relative to the ambient and conducting temperatures.

In some embodiments, a temperature is detected via the thermistor 500-2 when the respiration sensor 100a, 100b is initially placed on a patient's upper lip. Small sensors placed on the patient's airways or close to airways may block the airways if the sensor detaches or the attachment is loose. Some conventional approaches to mitigate the possibility of the sensor from detaching or becoming loose are to increase the size of the sensor and increase the adhesive area stuck to the skin. Larger sized sensors, however, may be uncomfortable for a patient and the increase in adhesive may irritate the skin of the patient, such that the patient may intentionally or unintentionally remove or detach the sensor. Some other conventional approaches may utilize a notification system when the sensor becomes detached. In such approaches, however, care personnel may experience "alarm fatigue" caused by false alarms. The disclosed respiration sensor 100a, 100b determines different suitable measurement parameters that are used to specify different situations to generate appropriate notifications.

For example, when the respiration sensor 100a, 100b is turned on, but is not yet placed on or attached to the patient's face, the thermistor 500-1, the thermistor 500-2, and the thermistor 400-3 detects ambient air temperature. Additionally, data for a breath indicator 1453a can be stored in a memory 1453b associated with the CPU 1444, and can indicate that no breaths have been detected yet by the thermistors 400. While the memory 1453b is illustrated to be included in the CPU 1444, it can be a separate element. When the respiration sensor 100a, 100b is placed on the patient's upper lip, the thermistor 500-2 comes into close contact with or makes contact with the skin of the upper lip and begins detecting the skin temperature on the patient's upper lip as represented by a skin temperature curve. For example, at zero seconds, the thermistor 500-2 detects the ambient air of approximately 23 °C and warms up after the respiration sensor 100a, 100b is placed on the upper lip of the patient, at approximately 8 seconds, to detect the skin temperature of approximately 35.5 °C at 55 seconds. Accordingly, when the respiration sensor 100a, 100b is removed or loosened from the skin on the patient's upper lip, the thermistor 500-2 adapts and begins detecting the ambient temperature.

In some embodiments, a temperature offset error may be induced to the thermistor 500-2 to compensate for any space between the thermistor 500-2 and the patient's upper lip, such as a mustache or similar medium. As a result, the temperature detected by the thermistor 500-2 may differ from the actual skin temperature. However, this compensation or adjustment may be tolerated as it may be important only to detect the temperature change. For example, when the respiration sensor 100a, 100b is placed on the patient's upper lip the thermistor 500-2 monitors or detects the temperature trend over time until detection of removal of the respiration sensor 100a, 100b rather than measuring or detecting the absolute skin temperature value.

In some embodiments, a temperature is detected via the thermistor 500-1 during ambient temperature change. The thermistor 500-1 monitors or detects the ambient temperature. For example, on an ambient temperature curve, the thermistor 500-1 detects an initial ambient temperature of approximately 23 °C at zero seconds and detects a new ambient temperature of approximately 25 °C at 55 seconds when, for example, the patient is transferred from an ambulance to a hospital environment.

In some embodiments, a temperature is detected via the thermistor 400-1, 400-2, 400-3 during respiratory flows. The thermistor 400-1, 400-2, 400-3 sequentially detects the temperature change of the breathing gas flow between exhaled breathing gas and inspired ambient air at a constant ambient temperature of 25 °C, as represented by a respiration temperature curve. During expiration, exhaled humid and warm air flows out from the nasal and/or the oral passages of the patient into the cavity, such as, for example, the nasal flow passages 301 and the oral flow passage 302, inside the sensor housing (e.g., housing 2001) causing temperature of the thermistor 400-1, 400-2, 400-3 located inside the cavity to adapt to the exhaled gas flowing past the thermistor 400-1, 400-2, 400-3. During inspiration, the patient inhales causing the ambient air to flow through the cavity, such as, for example, the nasal flow passages 301 and the oral flow passage 302, inside the sensor housing (e.g., housing 2001) towards the oral and/or nasal passages of the patient, at which point, the thermistor 400-1, 400-2, 400-3 adapts back to the temperature of inhaled ambient air flowing past the thermistor 400. Thus, during expiration, the air flowing out from the lungs warms up the thermistor 400-1, 400-2, 400-3 and, during inspiration, the ambient air cools down the thermistor 400-1, 400-2, 400-3. The temperature difference between the inhaled ambient air and the exhaled breathing gas decreases and approaches zero when the temperature of the ambient air approaches the temperature of the exhaled breathing gas. When the temperature of the inhaled ambient air exceeds the temperature of the exhaled breathing gas the temperature difference exceeds zero again, but changes its sign.

In some embodiments, continuous, real time measurements of respiratory flows is determined. Accordingly, a curve indicates a respiration real time waveform. Accordingly, the curve is a waveform including more than two breathing cycles, each cycle including an expiration phase (positive amplitude) and an inspiration phase (negative amplitude). A respiration rate (RR) curve is a curve indicating a value of breaths per minute [bpm]. It can be calculated from respiration waveform curve according to equation RR = 60 seconds/breathing cycle time [seconds]. Each respiration cycle has respiration magnitude that may be calculated from a difference between maximum amplitude of expiration and minimum amplitude of inspiration (which is negative). In some embodiments, respiration magnitude is proportional to a breathing flow rate. When a patient exhales, the warm, moist breathing gas from the lungs warm up thermistors 400-1, 400-2, 400-3 causing respiration waveform signal curve to rise. During inspiration, ambient air cools down thermistors 400-1, 400-2, 400-3 to a temperature close to the ambient air temperature. Thus, the breathing cycle amplitude is proportional to breathing gas flow rate or respiration magnitude, which is proportional to a temperature change of thermistors caused by the cooling/warming effects of inspiratory and expiratory air flowing past the thermistors. In the particular case of curve, respiration magnitude is a value in percentages indicating the breathing flow magnitude or rate, relative to a maximum breathing flow magnitude or a maximum rate for a particular patient.

In some embodiments, a respiration rate over an extended period of time may be monitored and fit to a curve. The curve may indicate a respiration magnitude, corresponding to the depth of breath, over an extended period of time. In some embodiments, curves may reflect both respiration rates and magnitude values calculated on a breath to breath basis. In some embodiments, the curves may include average values to reduce large fluctuations in signals received from sensors. In some embodiments, respiration rate and variance may be desirable parameters for detecting an upcoming heart stroke. In some embodiments, a breathing signal variance may anticipate a stroke event approximately 6-8 hours before the actual stroke. Similarly, overdose of opioids, or pain (e.g., too little opioids) may cause changes in respiration variance that are detectable in a respiration sensor, leading to quicker response and treatment to mitigate or prevent the impending risk.

### VI. Accelerometer Functions

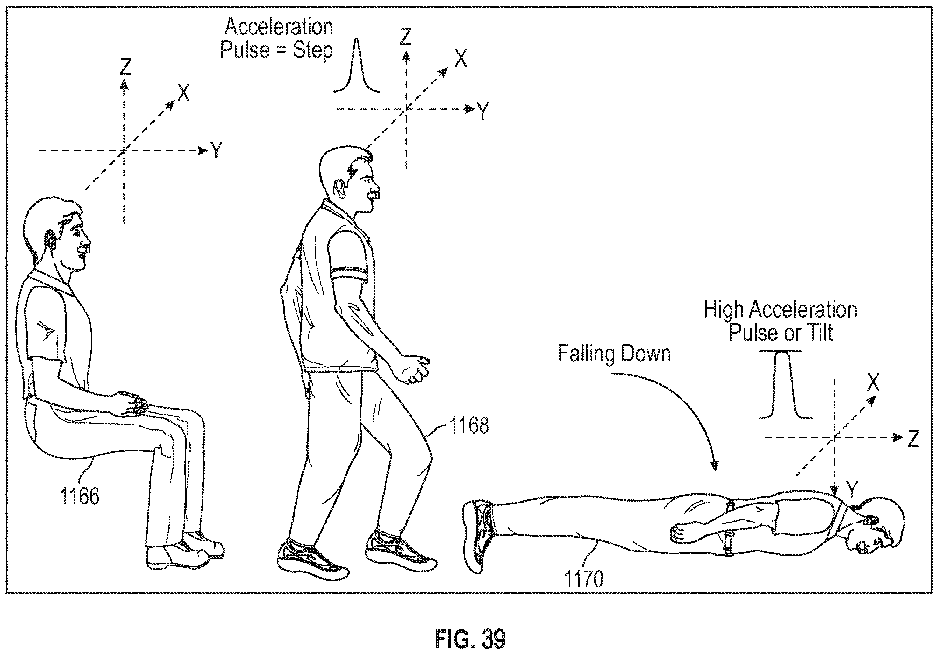

Referring to FIG. 39, the respiration sensor 100a, 100b can provide, for example, body position, movement, and fall detection via the accelerometer 1150 (shown in FIG. 33). The accelerometer 1150 measures or detects acceleration, position, angular rotation and other parameters derived from electrical signals proportional to at least x-, y-, and z-axes directions of accelerometer 1150 and can detect the patient's position and movement, the patient's head position and movement, acceleration caused by movement of the respiration sensor 100, 100b, 100c, and movement of patient's upper lip while talking or movement of the patient's heart. For example, the electrical signals from the accelerometer 1150 can be sent or transmitted to a monitoring device, such as a host monitor or similar client device, via Bluetooth or other communication method to monitor mobile patients. In some embodiments, the accelerometer 1150 of the respiration sensor 100a, 100b is a three-dimensional accelerometer that measures acceleration and position of at least x-, y-, and z-axes directions of the accelerometer 1150 as well as rotation around at least these three axes.

As discussed above, the respiration sensor 100a, 100b detects movement and position to monitor, for example, that the respiration sensor 100a, 100b has not fallen out of place with respect to the patient, that the patient has not fallen, or that the orientation of the patient's head is not obstructing the nasal and oral breathing gas flows (e.g., patient's face is downward towards pillow or bed). For example, it is desirable to obtain information about how a patient's head is positioned when the patient is lying in bed for determining the measurement of respiratory cycles from patients. When the patient is lying down on his/her back with his/her face upwards the patient can, for example, turn his/her head from left to right. In such a position, the patient can breathe in a manner that allows gas to flow freely through the nasal and/or oral cavities of the respiration sensor 100a, 100b. When the patient is lying sideways, his/her head can turn upward or downward. In this sideways position, it possible for the patient's head to face sideways or upward, such that the patient can breathe in a manner that allows gas to flow freely through the nasal and/or oral cavities of the respiration sensor 100a, 100b. It is also possible, however, in this sideways position, for the patient to turn his/her head downwardly toward the bed or a pillow, such that the gas does not flow freely or is obstructed through the nasal and/or oral cavities of the respiration sensor 100a, 100b. This uneven gas flow or obstruction of gas flow can disturb the measurement signal proportional to breathing or the patient's breathing may be prevented or deteriorated. A similar result may occur when the patient is laying on his/her stomach with his/her face downward into the bed or the pillow.

In such scenarios, the respiration sensor 100a, 100b may detect the direction in which the patient's face is pointing via the accelerometer 1150, which can also measure or detect the axial and/or angular position. The position of the patient's head is determined or calculated from the electrical signals in the x-, y-, and z-directions detected via the accelerometer 1150. In some embodiments, the respiration sensor 100a, 100b determines, via the signals proportional to the patient's position that are monitored by the accelerometer 1150, the position of the patient's head relative to the respiration sensor 100a, 100b. As a result, the respiration sensor 100a, 100b, responsive to determining that the patient's head is in a position that inhibits or obstructs gas flow therethrough and/or causes the respiration sensor 100a, 100b, to function improperly, can transmit a notification to inform of such positioning to the host monitor or other client device via Bluetooth or other communication method.

FIG. 39 illustrates the respiration sensor 100a, 100b in use on a patient to identify or detect any of a seated position 1166, a moving position 1168, and a fallen position 1170. In some embodiments, the respiration sensor 100a, 100b can identify or detect transitioning of a patient between any of a seated position 1166, a moving position 1168, and a fallen position 1170. In some scenarios, the patient may be mobile (e.g., getting up from the bed to use the restroom) and it may be desirable to monitor the patient's movement and position. For example, the patient may be recovering from a health issue and feel dizzy when getting up from a stationary position, such that the patient may pass out, fall down, or hurt himself/herself and require acute medical attention and care. In some embodiments, the respiration sensor 100a, 100b detects, via the signals proportional to the patient's position that are monitored by the accelerometer 1150, such situations and indicates or transmits a notification to inform or alert the host monitor or other client device via Bluetooth or other communication method.

As an example, the patient may be in a seated position 1166 and stand up to an upright position 1168, such that the accelerometer 1150 detects movement of the patient's head via the electrical signals in the x-, y-, and z-directions. Further, as the patient moves or walks in the upright position 1168, the accelerometer 1150 detects each step or movement the patient may make, such as when the patient gets out of bed to go to the restroom. Each step generates acceleration pulses that are detected by the accelerometer 1150 via the electrical signals proportional to acceleration in the x-, y-, z-directions. If the patient happens to fall down to the fallen position 1170, the accelerometer 1150 detects a high acceleration value proportional to a falling down magnitude. With the patient in the fallen position 1170 (e.g., lying on the floor) from the upright position 1168, the respiration sensor 100a, 100b determines that the patient has fallen down due to the accelerometer 1150 detecting a high acceleration value and determining the difference in the patient's head position in the upright position 1168 and the fallen position 1170. Responsive to the determination that the patient has fallen down, the respiration sensor 100a, 100b, transmits a notification to inform or alert the host monitor or other client device, via Bluetooth or other communication method, that the patient has fallen down and may require immediate medical care.

Additional measurements can be made based on movement of a patient's upper lip when patient talks. Talking is vibration of air coming from vocal cords and it may disturb the breathing gas flow measurement and the calculation of respiration rate (RR). The movement of the upper lip may be detected and indicate that the patient is talking. In some embodiments, movement of a patient's upper lip is detected by the accelerometer 1150.

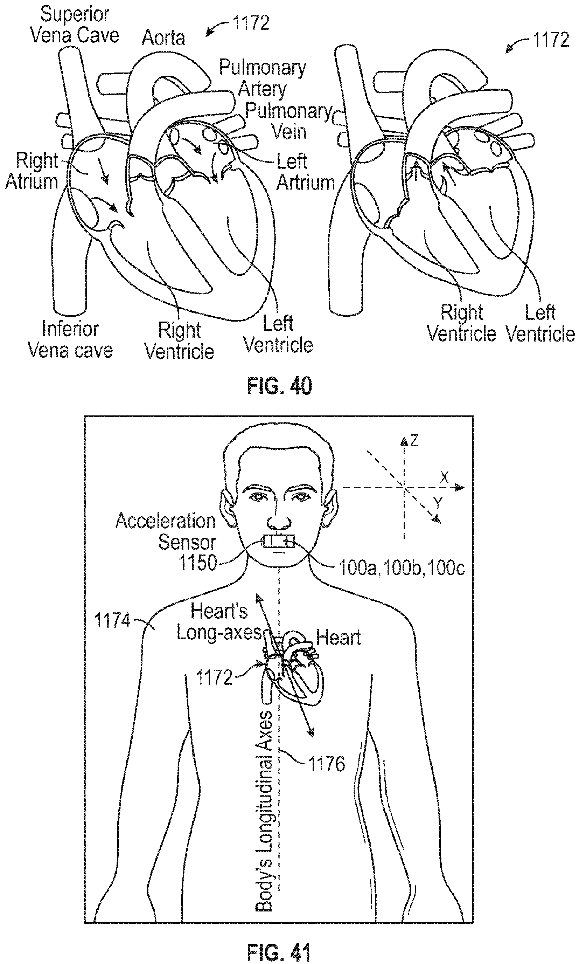

Additional measurement can be made based on movement of a patient's heart. The measurements can be used to determine a heart rate of the patient. FIG. 40 illustrates blood circulation through a heart 1172 as the heart 1172 pumps blood through the body 1174, shown in FIG. 41. Blood from the systemic circulation enters the right atrium from the superior and inferior vena cava and passes to the right ventricle. From the right ventricle, blood is pumped into the pulmonary circulation, through the lungs. Blood then returns to the left atrium, passes through the left ventricle and is pumped out through the aorta back to the systemic circulation. Normally, with each heartbeat, the right ventricle pumps the same amount of blood into the lungs as the left ventricle pumps to the body. Arteries transport blood away from the heart. The heart 1172 contracts at a resting rate close to 72 beats per minute.

Due to a specific orientation of the myocardial fibers, in a heartbeat cycle, the heart 1172 makes a wringing or twisting motion along its long-axis. On the other hand, the heart's sequential contraction, which allows superior and inferior blood to enter the right atrium and ventricle as well as allows expansion to pump blood from the left ventricle and the atrium back to the systemic and pulmonary blood circulation, generate micro movement along heart's long-axis. This back and forth movement is slightly leaned to the right regarding the body's longitudinal axis 1176, as illustrated in FIG. 41.

The heart's movement moves the whole body 1174 back and forth cyclically at the phase of a heartbeat close to the direction along body's longitudinal axis 1176. This micro movement can be detected by the accelerometer 1150 of the respiration sensor 100a, 100b. The most sensitive direction for the accelerometer 1150 to detect would be the z-axis. In some embodiments, the accelerometer 1150 contains an angular motion sensor or sensing elements, in addition or alternatively, such that it can be used to detect the heart's rotation along its long-axis, which also generates rotational force around body's longitudinal axis 1176 at a phase of the heartbeat. Either or both the body's longitudinal movement or rotational movement around the body's longitudinal axis 1176 can be transformed to a heartbeat or heartbeats per minute value from the electrical signals of accelerometer. This heart rate (HR) information can be used together with the respiration rate (RR) and flow information, by the respiration sensor 110a, 100b, in early detection and prevention of respiratory depression and other symptoms.

In some embodiments, the accelerometer 1150 can also detect rise and fall of a patient's chest or other thoracic movement. This information can be coupled with at least one of HR, RR, and other breath indicators to aid in early detection and prevention of respiratory distress and other illnesses.

### VII. EtCO2 Surfaces

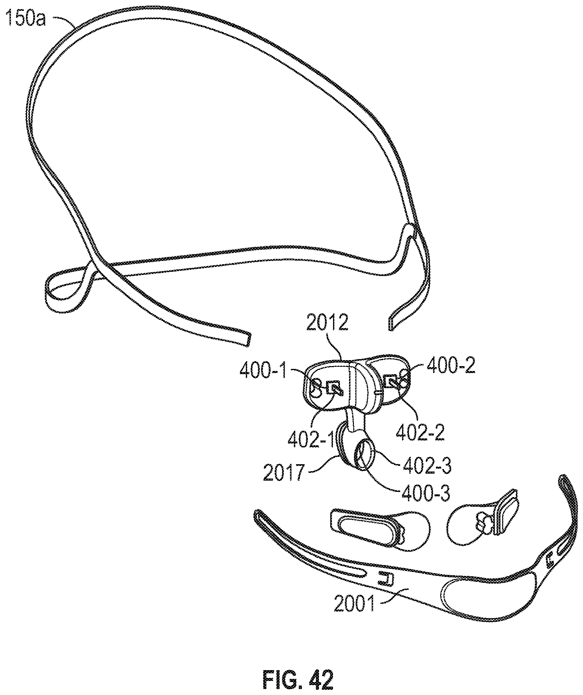

In some embodiments of the present disclosure, the respiration sensor, such as, for example, the respiration sensor 100a, 100b, can include end-tidal CO2 (EtCO2) sensing features. The EtCO2 sensing features can include one or more EtCO2 sensitive surface. The one or more EtCO2 sensitive surface can be positioned on an outer surface of the shroud 2012 and on a surface of the oral shroud 2017. FIG. 42 shows a first EtCO2 sensitive surface 402-1 positioned on an outer surface of the shroud 2012 and adjacent to the thermistor 400-1, a second EtCO2 sensitive surface 402-2 positioned on an outer surface of the shroud 2012 and adjacent to the thermistor 400-2, and a third EtCO2 sensitive surface 402-3 positioned on an inner surface of the oral shroud 2017 and adjacent to the thermistor 400-3.

The EtCO2 sensitive surface can change color as a result of nasal and/or oral breath detection of CO2. For example, the EtCO2 sensitive surface can change color to indicate the presence of CO2. In some embodiments, the one or more EtCO2 sensitive surface is coupled with an electrode. As nasal and/or or oral breath moves over the EtCO2 sensitive surface, a change in resistance can occur. The change in resistance is used to determine the presence of CO2 or other breathing related conditions.

### VIII. Interconnectivity

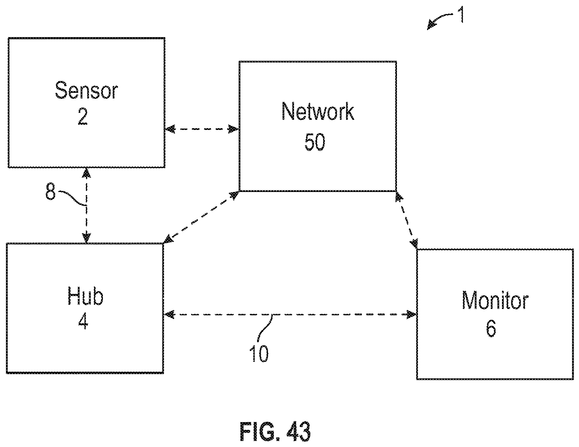

Referring to FIG. 43, a respiration monitoring system 1 is illustrated including a sensor 2, such as respiration sensor 110a, 100b, a hub 4, and a monitor 6. The sensor 2, hub 4, and monitor 6 can be in communication with each other with wires or wirelessly. In some embodiments, any of a sensor 2, a hub 4, and a monitor 6 can be in communication with each other and with a network 50. The network can include, for example, any of a local area network (LAN), a wide area network (WAN), the Internet, a remote or cloud server, and the like. Further, network 50 can include, but is not limited to a network topologies, including any of a bus network, a star network, a ring network, a mesh network, a star-bus network, tree or hierarchical network, and the like. Although one sensor 2, hub 4, and monitor 6 are shown, it should be understood that the respiration monitoring system can include multiple sensors 2, hubs 4, and monitors 6.

Some embodiments of the respiration monitoring system can include a patient inside a hospital, a patient at home (e.g., homecare), and other original equipment manufacturer (OEM) applications. Accordingly, in some embodiments OEM parameters can be added to monitoring system (i.e., SpO2, Temp, NiBP, ECG etc.)

Communication between the sensor 2 and any of a hub 4 and a monitor 6 can be established using low energy communication 8, such as Bluetooth. A hub near the respiration sensor, for example, attached to or near a patient, can enable longer respiration sensor operation time by using low energy communication 8. The low energy communication 8 can include any of a wireless personal area network technology or Bluetooth. The hub can also provide respiration sensor pairing with patient, which can help secure patient identification information. Further, the use of a hub 4 with the sensor 2 can permit patient mobility and continuous monitoring throughout the hospital.

A long distance communication 10 protocol (e.g., Wi-Fi, cellular or other communication) may provide data transfer between the hub 4 and a monitor 6. In some embodiments, data can transfer between the hub 4 and a monitor 6 through the network 50. In some embodiments, the sensor 2 communicates with a hub in the form of a smartphone. The smartphone communicates to internet through Wi-Fi or cellular systems. Data can be transferred and saved into a cloud in real time. Patient data can be viewed in a different physical location in real time with a smartphone, a tablet, a laptop or desktop computer, a smart TV, and the like.

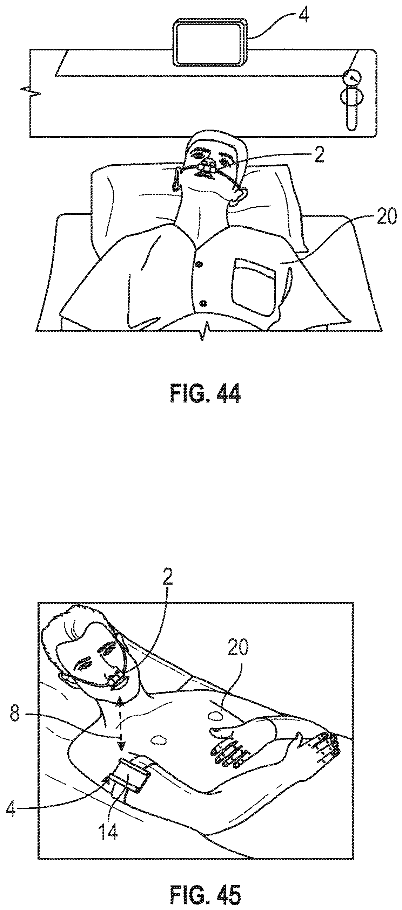

FIG. 44 illustrates a sensor 2, such as respiration sensor 110a, 100b, coupled to a patient's 20 head, and a hub 4 positioned adjacent to the patient. The hub 4 provides a user interface to the clinician for bedside monitoring. The hub 4 can also provide connectivity and communication between the patient 20 and a network 50 of the hospital.

FIG. 45 illustrates a sensor 2, such as respiration sensor 110a, 100b, coupled to a patient's 20 head, and a hub 4, in the form of a smartphone 14 connected via a band to the patient's 20 arm. The hub 4 provides a user interface to the clinician for bedside monitoring. The hub 4 can also provide connectivity and communication between the patient 20 and a network 50 of the hospital. In some embodiments of the present disclosure, the smartphone 14 can be placed on a holder adjacent to the patient. The holder can couple with the smartphone 14 to provide any of a communication interface of charging of the smartphone 14.

The smartphone 14 may include a camera, which can be used for pairing with the sensor 2; Bluetooth to communicate with a low power consumption sensor 2; Wi-Fi to communicate with cloud & hospital network; a user interface enabled for a patient and/or a caregiver; 4G, WCDMA, and GPS. In some embodiments, the smartphone 14 communication is disabled for in-hospital use, and enabled for out-of-hospital use. For example, in out-of-hospital use, when patient and user authentication may be less readily available, the smartphone 14 may perform a face recognition algorithm or other personal/visual/audible recognition algorithms to pair the patient 20 and the respiration sensor 2, and authenticate that the pairing is correct and accurate. When any information is not authenticated, smartphone 14 may issue an alert, sound an alarm, or communicate a warning to a nurse in the centralized system. In some embodiments, the smartphone 14 is configured to integrate with hospital system to provide authentication of patient and/or user during in-hospital use.

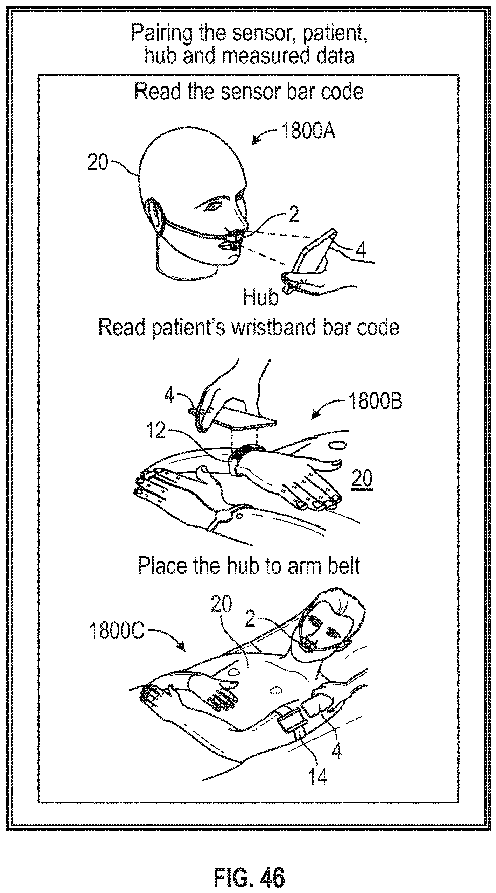

FIG. 46 illustrates an interaction between, for example, the sensor 2 and the smartphone 14 in a respiration monitoring system, according to some embodiments. The interaction can be used to pair the sensor 2 and the hub 4, and can be used to identify the patient with the sensor 2.

In a first step 1800A, a nurse or authorized healthcare personnel may read data from the sensor 2 in a proximity mode (e.g., a sensor identification value, such as a barcode and the like). In a second step 1800B, the healthcare personnel may further read the patient's wristband 12 to log in the respiratory data in the appropriate patient record. In a third step 1800C, the healthcare personnel may securely place the smartphone 4 in an arm belt 14 on the patient 20. After connection of the hub 4 with a network 50 or a centralized server, for example, the sensor 2 can send and/or receive, in real-time, continuous respiratory data and other information to the network 50 or centralized server.

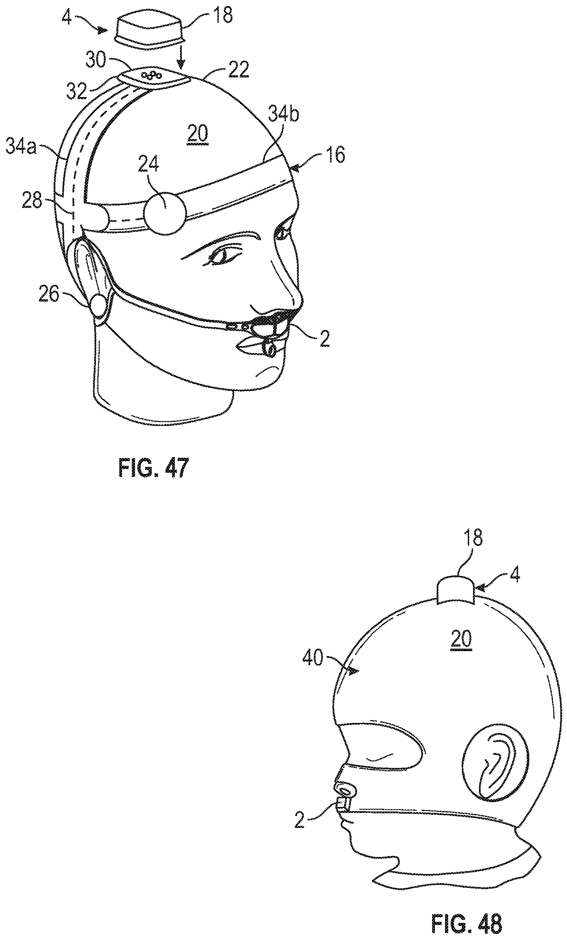

FIG. 47 illustrates a sensor 2, such as respiration sensor 110a, 100b, and a headdress 16 coupled to the head of a patient 20. The headdress 16 provides an easy to wear, wireless monitoring structure for a mobile patient. The headdress includes a hub 4, in the form of a pod 18 that can be coupled to the headdress 16 at a position adjacent the top of the patient's head.

The headdress 16 can contain sensors attached to, integrated into or in connection with headdress fabric. A sensor 2 (e.g., respiration sensor 100a, 100b) can measure respiration rate and flow. The pod 18 can include a pod sensor 22 to measure any of skin temperature, ambient temperature, or position, motion and acceleration of the patient.

A head sensor 24 can be configured to engage against the patient's head when the headdress is worn by the patient. In some embodiments, the head sensor 24 is positioned adjacent to the temple of the patient's head when the headdress is worn by the patient. In some embodiments, the head sensor 24 can extend across the patient's forehead. The head sensor can measure any of temperature, frontal EEG, frontal oxygen saturation, or movement of the patient. In some examples, the head sensor 24 includes electrodes positioned at different positions on the patient's head to measure full EEG.

An ear sensor 26 can be configured to engage against an ear lobe of the patient when the headdress is worn by the patient. The ear sensor 26 can measure oxygen saturation. The sensor 2 and headdress sensors 22, 24, 26 can transform physiological signals into electrical signals for measuring physiological parameters. For example, respiration sensor 110a, 100b, and/or other headdress sensors 22, 24, 26, can measure any of respiration rate (RR), breathing gas flow, nasal-SpO2, ear-SpO2, frontal-SpO2, pulse rate (PR), heart rate (HR), skin temperature, ambient temperature, core temperature, body position or movement, chest or thoracic motion, EtCO2, full-EEG, frontal EEG, or similar parameters. The sensors are located at suitable locations around the headdress, depending on the measured physical parameter, to enable optimized measurement of that parameter.

Each sensor may contain a battery to electrically power up the sensor and each sensor may also contain a transceiver to communicate with a host (e.g., network 50) or monitor further away. Preferably sensors are electrically powered through wires 28 integrated into headdress 16, which connect the sensors with a battery located into one location on the headdress 16. The sensors also communicate with the host through one transceiver located in the pod 18. The data communication between the sensors and the transceiver can be via the wires 28 integrated into headdress. This simplifies the electronics and power management infrastructure, decreases radio frequency pollution, which improves communication quality, lowers the cost, weight and size, decreases the power consumption and improves usability and patient comfort.

The sensors attached to headdress 16 only contain a minimum amount of mechanics and electronics to simplify and minimize the sensors infrastructure. For example, to enable the measurement of a physiological signal, only the parameter specific electronics to enable to transform the physiological signal of that specific parameter into an electrical signal are located into each sensor. All the electronics that have commonalities between the sensors can be combined in the pod 18, which can also include the battery, processing unit, transceiver and similar. This centralizing reduces complexity, makes size and weight smaller, increase patient comfort and usability and also reduces the cost of the sensors.

Sensors located on fixed or certain places on the headdress 16 also increase the usability and the quality of measurement as sensors locate and place optimally on patient's head regardless of patient's appearance or differences between users. Simpler, easy to dress wearable system also increases the adoption of a complex multi-parameter system.

The pod 18 can be removed for reuse, and the headdress 16 and sensors therein disposed. Disposability reduces cross contamination risk and decreases care personnel's working time needed for otherwise disinfecting products.

The pod 18 can include most of the electronics, radio transceiver, electrical power source such as a battery, processor etc. and software. The system hardware and mechanics are simplified by centralizing complex functions into a reusable pod, which also makes the system more efficient, easy to clean to prevent cross contamination between patients and low cost. Further, the top of the head is also one of the most comfortable places for the pod 18 when patient is lying, sitting or moving, but it also ensures easy device access and alarm visibility to care personnel.

Electrical signals from any of the headdress sensors 22, 24, 26 and respiration sensor 2 can be transmitted from through the electrical wires 28 to the pod 18 where they are processed into suitable form to be transmitted wirelessly to the monitor. In some embodiments, the pod 18 can communicate with any of the sensors 2, 22, 24, 26, headdress 16, and the monitor via Low energy Bluetooth or similar communication method. Preferably the communication with the monitor is via WiFi, 3G, 4G communication or similar. This ensures that data from a mobile patient can be transferred to a monitor device and hospital from any place inside or outside hospital.

To ensure data is not lost during communication interruption the pod 18 can contain internal First in first out (FiFo) memory to record data for a time of interruption. The monitor shows the processed data in a suitable form, for example on the host's display in digits and waveforms and alarms the care personnel when needed.

The pod 18 can have electrical contacts on a surface, which are configured to engage against reciprocal electrical contacts 30 on a surface of a pod frame 32 coupled to the headdress 16. In some embodiments, the electrical contacts 30 in connection with the headdress 16 are planar. When pod 18 is attached to pod frame 32 these electrical contacts 30 connect electrical power and electrical data lines to enable power and data transfer between the sensors 2, 22, 24, 26 and the pod 18 through the electrical wires integrated into to headdress 16. The attachment between the pod 18 and pod frame 32 may be mechanical sliding or pressing into rails or it may be magnetic or similar.

A battery inside the pod 18 can be rechargeable. When charging is needed, the pod 18 can be separated from the pod frame 32 and coupled to a source of electricity. In some embodiments, the pod 18 can be placed on a wireless charging table or a docking station based on for example inductive charging.

The outer surfaces of pod 18 can be smooth to prevent injury, prevent catching on fabric, and permit easy cleaning and disinfecting. Power on/off and similar functions are implemented with for example capacitive buttons rather than mechanical buttons so that the user only touches the marked areas on the surfaces of pod 18. The pod 18 can have any of an alarm light and an audible alarm. The alarm light or audible alarm can be integrated inside the pod 18. The alarm light can become visible through a partially transparent housing made of material such as plastic.

The headdress can include straps 34a, 34b that extend around at least a portion of the patient's 20 head, as illustrated in FIG. 47. The headdress 16 can be configured so that, when the headdress 16 is worn by a patient 20, a first strap 34a can extend over the top of the patient's head, and a second strap 34b can extend across the forehead of the patient. The headdress 16 can include a fastener to permit attachment of the straps 34a, 34b to each other and to adjust the headdress 16 to conform to a particular patient's head. The fastener can include any of a hook and loop fastener, button, snap, or adhesive. In some embodiments, the at least a portion of the straps 34a, 34b or headdress 16 is formed of an elastic material.

FIG. 48 illustrates an embodiment of a headdress 40, which extends along a greater portion of the patient's 20 head relative to the headdress 16 illustrated in FIG. 47. When worn by a patient 20, the headdress 40 can extend along any of the patient's head top, forehead, crown, and nape, as well as the upper lip. The additional area covered by the headdress 40 distributes pressure against the patient 20 over a greater area, thereby reducing discomfort. Further, the additional area covered by the headdress 40 can resist movement of the headdress 40 relative to the patient's head. The headdress 40 can be used for adults and/or children as well as infants.

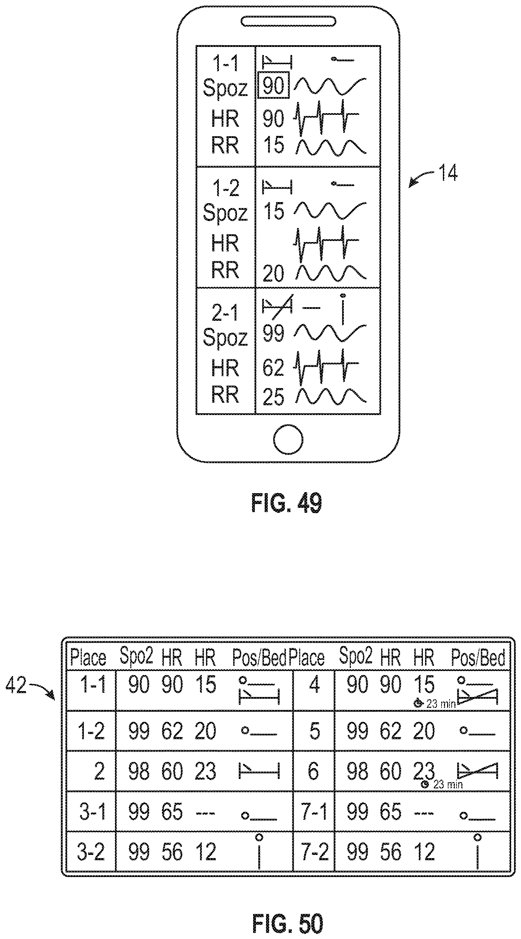

Referring to FIGS. 49 and 50, examples of monitors are illustrated. The monitor can be any device or system where data is received from a hub 4 or respiration sensor 2. FIG. 49 illustrates a monitor in the form of a smartphone 14. The smartphone 14 can be a patient's phone, a caregiver's phone, or the phone of another person monitoring the patient. FIG. 50 illustrates a monitor in the form of a central station 42. The central station 42 can be a television, computer station, display board, or another display that can be observed by a person monitoring the patient.

The monitor can graphically display information regarding the patient and/or data received from any of the sensor 2 and hub 4. The displayed information can include a temperature value from at least one of two nasal flow passages, a temperature value from an oral flow passage, a temperature value of a patient's skin surface, and a temperature value of a patient's environment. In some embodiments, the displayed information includes an identification of the patient and/or their location (e.g., 1-1, 1-2, 2-1), SpO2 measurement, heart rate, and respiration rate. Additionally, displayed information can include an indication of a patient's orientation or position. The patient's orientation or position can be shown in text or as a symbol. For example, the text or symbol may represent whether the patient is lying on the bed, is standing upright, is sitting up, or is in some other position.

### Illustration of Subject Technology as Clauses

Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identifications.

Clause 1. A respiration sensor comprising: a housing having a first nasal flow passage and a second nasal flow passage that extend therethrough, wherein the first and second nasal flow passages are disposed in parallel to one another with respect to a nasal respiratory flow direction; and an electronics board comprising a first nasal thermistor and a second nasal thermistor, the electronics board coupled to the housing such that the first and second nasal thermistors are positioned into each of the first and second nasal flow passages, respectively.

Clause 2. The respiration sensor of Clause 1, wherein the electronics board comprises a support structure, the support structure having a proximal portion coupled to the electronics board and a distal portion transverse to a plane defined by a top of the electronics board, wherein, when the electronics board is positioned within the housing, the distal portion of the support structure extends into at least one of the first and second nasal flow passages.

Clause 3. The respiration sensor of Clause 2, wherein any of the first or second nasal thermistors are coupled to the distal portion of the support structure.

Clause 4. The respiration sensor of any of Clauses 1 and 2, further comprising an oral flow passage and an oral thermistor, the oral flow passage disposed transverse to the first and second nasal flow passages, along an oral respiratory flow direction.

Clause 5. The respiration sensor of Clause 4, wherein the electronics board comprises a support structure having a proximal portion coupled to the electronics board and a distal portion extending along a plane defined by a top of the electronics board wherein, when the electronics board is positioned within the housing, the distal portion of the support structure extends into at least one of the first and second nasal flow passages.

Clause 6. The respiration sensor of Clause 5, wherein the oral thermistor is coupled to the distal portion of the support structure.

Clause 7. The respiration sensor of any of Clauses 4 to 6, further comprising at least one oral flow guide disposed in the oral flow passage.

Clause 8. The respiration sensor of Clause 7, wherein a first oral flow guide of the at least one oral flow guide is disposed proximate an oral inlet of the oral flow passage and a second oral flow guide of the at least one oral flow guide is disposed proximate an oral outlet of the oral flow passage.

Clause 9. The respiration sensor of Clause 8, wherein any of the oral inlet and the oral outlet is elliptical.

Clause 10. The respiration sensor of any of Clauses 8 and 9, wherein the oral flow passage tapers from the oral inlet toward the oral outlet.

Clause 11. The respiration sensor of any of Clause 1 to 10, further comprising a third thermistor and a fourth thermistor, wherein the third thermistor is an ambient thermistor and the fourth thermistor is a skin thermistor configured to determine whether the respiration sensor is properly positioned against a patient's physiognomy.

Clause 12. The respiration sensor of Clause 11, wherein the electronics board further comprises a filter configured to subtract a first electrical signal detected by the skin thermistor from a second electrical signal detected by the ambient thermistor.

Clause 13. The respiration sensor of any of Clauses 11 and 12, wherein the electronics board further comprises a filter configured to subtract a first electrical signal detected by the skin thermistor and a second electrical signal detected by any of the first and second nasal thermistors and an oral thermistor from a third electrical signal detected by the ambient thermistor.

Clause 14. The respiration sensor of any of Clauses 1 to 13, further comprising a shroud configured to protect the electronics board and to form at a least a portion of the first and second nasal flow passages.

Clause 15. The respiration sensor of any of Clauses 1 to 14, wherein the electronics board further comprises an accelerometer configured to detect movement of the respiration sensor.

Clause 16. The respiration sensor of Clause 15, wherein the accelerometer is configured to determine whether the respiration sensor has fallen from a face of a patient or the patient has fallen.

Clause 17. The respiration sensor of any of Clauses 1 to 16, wherein the electronics board further comprises a radio transceiver configured to communicate with an external device that is coupled with a network.

Clause 18. The respiration sensor of any of Clauses 2 to 17, wherein the electronics board comprises a capacitive sensor configured to detect a contact between the housing and a patient's face, when the respiration sensor is in condition for use.

Clause 19. The respiration sensor of any of Clauses 1 to 18, further comprising at least one nasal flow guide disposed in each of the first and second nasal flow passages.

Clause 20. The respiration sensor of Clause 19, wherein a first nasal flow guide of the at least one nasal flow guide is disposed proximate a nasal inlet of one of the first and second nasal flow passages, and a second nasal flow guide of the at least one nasal flow guide is disposed proximate a nasal outlet of the one of the first and second nasal flow passages.

Clause 21. The respiration sensor of any of Clauses 1 to 20, further comprising a battery.

Clause 22. A respiration sensor comprising: one or more thermistors configured to detect at least one of an inspiratory temperature, an expiratory temperature, an ambient temperature adjacent the respiratory sensor, or a temperature of a patient's skin engaged against the respiration sensor; an accelerometer configured to detect at least one of a movement of the patient, a position of the patient, a heart rate, or a respiration rate; and an electronics board coupled to the one or more thermistors and the one or more thermistors.

Clause 23. The respiration sensor of Clause 22, comprising a thermistor configured to detect a temperature of a patient's skin engaged against the respiration sensor.

Clause 24. The respiration sensor of Clause 22, comprising a EtCO2 sensitive surface configured to detect the presence of CO2.

Clause 25. A system, comprising: a server having a memory storing commands, and a processor configured to execute the commands to: receive, from a hub, a data indicative of a respiratory condition of a patient; transfer the data into a memory in a remote server; provide the data to a mobile computer device, upon request; and instruct the mobile computer device to graphically display the data, wherein the data comprises a temperature value from at least one of two nasal flow passages, a temperature value from an oral flow passage, a temperature value of a patient's skin surface, and a temperature value of a patient's environment.

Clause 26. The system of Clause 25, wherein the processor is configured to determine a respiration rate from the data indicative of the respiratory condition of a patient.

Clause 27. The system of any of Clauses 25 to 26, wherein the processor is configured to determine a respiration magnitude from the data indicative of the respiratory condition of a patient.

Clause 28. The system of any of Clauses 25 to 27, wherein the processor is configured to determine a probability of a patient having a stroke or the patient being under an opioid based on a variance of the data indicative of the respiratory condition of a patient.

Clause 29. A method, comprising: receiving, from a hub, a data indicative of a respiratory condition of a patient; transferring the data into a memory in a remote server; providing the data to a monitor, upon request; and instructing the monitor to graphically display the data, wherein the data comprises a temperature value from at least one of two nasal flow passages, a temperature value from an oral flow passage, a temperature value of a patient's skin surface, and a temperature value of a patient's environment.

Clause 30. The method of Clause 29, further comprising determining a respiration rate from the data indicative of the respiratory condition of a patient.

Clause 31. The method of any of Clauses 29 to 30, further comprising determining a respiration magnitude from the data indicative of the respiratory condition of a patient.

Clause 32. The method of any of Clauses 29 to 31, further comprising determining a probability of a patient having a stroke or the patient being under an opioid based on a variance of the data indicative of the respiratory condition of a patient.

Clause 33. The method of any of Clauses 29 to 32, further comprising associating, in the remote server, a patient record with the respiratory condition of the patient.

Clause 34. The method of any of Clauses 29 to 33, wherein the patient is one of a hospital patient or a home-care patient, the method further comprising alerting an emergency care unit when the respiratory condition of the patient indicates a catastrophic event.

Clause 35. A respiration sensor system comprising: a respiration sensor comprising a housing having a nasal flow passage that extends therethrough, wherein the nasal flow passage is aligned with a nasal respiratory flow direction, and an electronics board comprising a nasal thermistor, the electronics board coupled to the housing such that the nasal thermistor is positioned into the nasal flow passage; and a hub configured to move data between the respiration sensor and a network.

Clause 36. The respiration sensor system of Clause 35, wherein the hub is a smartphone.

Clause 37. The respiration sensor system of Clause 35, further comprising a monitor configured to receive data from an of the respiration sensor and the hub.

Clause 38. A method, comprising: monitoring data using a respiration sensor; receiving, by a hub separate from the respiration sensor, data from a respiration sensor; transmitting, from the hub to a network, the data from the respiration sensor; and transmitting, from the network to a monitor, the data from the respiration sensor.

Clause 39. The method of Clause 38, wherein the data monitored by the respiration sensor is monitored via at least one of a skin thermistor, an ambient thermistor, at least one nasal thermistor, an oral thermistor, an accelerometer, and a breath indicator.

Clause 40. The method of Clause 39, further comprising receiving, by the hub from the respiration sensor, a notification indicating a correctly-placed-no-breath state, wherein receiving the notification is in response to the respiration sensor determining that the skin thermistor is detecting a skin temperature, the ambient thermistor is detecting an ambient air temperature, one of the at least one nasal thermistor and the oral thermistor is detecting the ambient air temperature, and the breath indicator is detecting breaths.

Clause 41. The method of any of Clauses 39 to 40, further comprising receiving, by the hub from the respiration sensor, a notification indicating a loose state, wherein receiving the notification is in response to the respiration sensor determining that the skin thermistor is detecting an ambient air temperature, the ambient thermistor is detecting the ambient air temperature, one of the at least one nasal thermistor and the oral thermistor is detecting a gas flow temperature, and the breath indicator is detecting breaths.

Clause 42. The method of any of Clauses 39 to 41, further comprising receiving, by the hub from the respiration sensor, a notification indicating any of a detached or no breath state, wherein receiving the notification is in response to the respiration sensor determining that the skin thermistor is detecting an ambient air temperature, the ambient thermistor is detecting the ambient air temperature, one of the at least one nasal thermistor and the oral thermistor is detecting the ambient air temperature, and the breath indicator is detecting no breaths.

Clause 43. The method of any of Clauses 39 to 42, further comprising receiving, by the remote hub from the respiration sensor, a notification indicating an operating temperature exceeded state, wherein receiving the notification is in response to the respiration sensor determining that the skin thermistor is detecting a skin temperature and the ambient thermistor is detecting a temperature equal to or greater than the skin temperature.

### Further Consideration

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

As used herein, the phrase "at least one of' preceding a series of items, with the term "and" or "or" to separate any of the items, modifies the list as a whole, rather than each member of the list (i.e., each item). The phrase "at least one of' does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Furthermore, to the extent that the term "include," "have," or the like is used in the description or the claims, such term is intended to be inclusive in a manner similar to the term "comprise" as "comprise" is interpreted when employed as a transitional word in a claim. The word "exemplary" is used herein to mean "serving as an example, instance, or illustration." Any embodiments described herein as "exemplary" is not necessarily to be construed as preferred or advantageous over other embodiments.

In one or more aspects, the terms "about," "substantially," and "approximately" may provide an industry-accepted tolerance for their corresponding terms and/or relativity between items.

A reference to an element in the singular is not intended to mean "one and only one" unless specifically stated, but rather "one or more." The term "some" refers to one or more. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

While certain aspects and embodiments of the subject technology have been described, these have been presented by way of example only, and are not intended to limit the scope of the subject technology. Indeed, the novel methods and systems described herein may be embodied in a variety of other forms without departing from the spirit thereof. The accompanying claims and their equivalents are intended to cover such forms or modifications as would fall within the scope and spirit of the subject technology.

## Claims

1. A respiration sensor system comprising:
a headdress (16) comprising a hub (4)
a respiration sensor (2) coupled to the headdress, the respiration sensor comprising one or more thermistors and an accelerometer, the one or more thermistors configured to detect at least one of an inspiratory temperature, an expiratory temperature, an ambient temperature adjacent the respiratory sensor, or a temperature of a patient's skin engaged against the respiration sensor, and the accelerometer configured to detect at least one of a movement of the patient, a position of the patient, a heart rate, or a respiration rate; and
an electronics board coupled to the one or more thermistors and the accelerometer and configured to measure any of respiration rate (RR), breathing gas flow, a nasal-SpO2, ear-SpO2, frontal-SpO2, pulse rate (PR), heart rate (HR), skin temperature, ambient temperature, core temperature, body position or movement, chest or thoracic motion, EtCO2, full-EEG, and frontal EEG.

2. The respiration sensor of Claim 1, wherein the headdress (16) comprises a headdress sensor (22, 24, 26) attached thereto.

3. The respiration sensor of Claim 2, wherein the headdress sensor (22, 24, 26) comprises a head sensor (24) configured to engage against the head of the patient when the headdress is worn by the patient.

4. The respiration sensor of Claim 2, wherein the headdress sensor (22, 24, 26) comprises an ear sensor (26) configured to engage against an ear lobe of the patient when the headdress is worn by the patient.

5. The respiration sensor of Claim 2, wherein the headdress comprises electrical wires configured to transmit a signal between any of the respiration sensor (2) and the headdress sensor (22, 24, 26).

6. The respiration sensor of Claim 2, wherein a signal is transmittable between any of the respiration sensor (2), the headdress sensor (22, 24, 26), and the hub (4) through Low energy Bluetooth or similar communication method.

7. The respiration sensor of Claim 1, wherein the electronics board is positioned in the hub (4) and is coupled to the one or more thermistors, the accelerometer, and the headdress sensor (22, 24, 26).

8. The respiration sensor of Claim 1, wherein the electronics board comprises any of a battery, processing unit, and transceiver.

9. The respiration sensor of Claim 1, wherein the hub (4) comprises a pod (18) that is removably coupled to a pod frame (32).

10. The respiration sensor of Claim 9, wherein the pod (18) comprises electrical contacts configured to engaged against electrical contacts (30) on a surface of the pod frame (32).

11. The respiration sensor of Claim 1, wherein the hub (4) is positioned adjacent to a top of a head of a patient when the headdress (16) is worn by the patient.

12. The respiration sensor of Claim 1, wherein the respiration sensor (2) comprises housing (2001) having a nasal flow passage (301, 2018) that extends therethrough, and the one or more thermistors are positioned in the nasal flow passage (301, 2018).

13. The respiration sensor of Claim 1, wherein the one or more thermistors comprises a nasal thermistor (400-1, 400-2).

14. The respiration sensor of Claim 1, wherein the one or more thermistors comprises an ambient thermistor (500-2) and a skin thermistor (500-2) configured to determine whether the respiration sensor is properly positioned against a patient's physiognomy.

15. The respiration sensor of Claim 1, comprising an EtCO2 sensitive surface configured to detect a presence of CO2.
